# EUROPEAN PATENT APPLICATION

(11) **EP 1 650 308 A1**
(43) Date of publication of application: **26.04.2006**
(21) Application number: 04021064.3
(22) Date of filing: 03.09.2004
(51) Int. Cl.: C12N 15/86, C07K 14/165, C07K 14/17, A61K 39/395, C07K 14/14, A61K 39/15

(54) **Nucleic acid sequences encoding proteins capable of associating into a virus-like particle**

(71) Applicant: CONSEJO SUPERIOR DE INVESTIGACIONES CIENTIFICAS, 28006 Madrid (ES); Fort Dodge Veterinaria S.A., 17813 Vall de Bianya (Girona) (ES)
(72) Inventor: Ceriani, Juan Eduardo, Dr., Olot, Gerona (ES); Ortego, Francisco Javier, Dr., 28025 Madrid (ES); Ejuanes Sanches, Luis, 28109 Madrid (ES); Plana Dúran, Juan, 17811 Santa Pau, Girona (ES)
(74) Representative: UEXKÜLL & STOLBERG

(57) **Abstract**

The present invention relates to nucleic acids comprising:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of a different virus, which is not FMDV, wherein the one or more proteins are capable of associating into a virus-like particle (VLP) that does not contain any infectious nucleic acid.

The present invention further relates to vectors, virus particles and host cells comprising these nucleic acids as well as their use for the preparation of vaccines, specifically for the preparation of vaccines.

SEQUENCE LISTING

<110> Consejo Superior de Investigaciones Cientificas

<120> Nucleic Acid Sequence encoding Proteins capable of associating into
a Virus-like Particle

<130> P 66673

<160> 19

<170> PatentIn version 3.1

<210> 1
<211> 2643
<212> DNA
<213> Rotavirus sp.

<400> 1
   atggcgtaca ggaaacgtgg agcgcgccgt gaggcgaata taaataataa tgaccgaatg 60
   caagagaaag atgacgagaa acaagatcaa aacaatagaa tgcagttgtc tgataaagta 120
   ctttcaaaga aagaggaagt cgtaaccgac agtcaagaag aaattaaaat tgctgatgaa 180
   gtgaagaaat cgacgaaaga agaatctaaa caattgcttg aagttttgaa aacaaaagaa 240
   gagcaccaaa aagagataca atatgaaatt ttgcaaaaaa cgataccaac atttgaacca 300
   aaagagtcaa tattgaaaaa attggaggat atcaaaccgg aacaagcgaa gaagcagact 360
   aagctattta gaatatttga accgagacag ctaccaattt atagagcgaa tggtgaaaaa 420
   gagttgcgta acagatggta ttggaagctg aagaaagata ctttaccaga tggagattat 480
   gatgttagag aatactttct aaatttgtat gatcaggttc ttactgaaat gccagattat 540
   ttactattaa aagatatggc agttgaaaat aaaaattcga gagatgccgg taaagttgtt 600
   gattctgaaa cagcaagtat ctgtgatgct atatttcaag atgaggaaac agaaggtgca 660
   gtgagacgat tcattgcgga gatgagacag cgcgtacaag ctgacagaaa cgttgtcaat 720
   tacccatcaa tattgcatcc aatagattac gcttttaatg agtatttttt gcaacaccaa 780
   ttagttgaac cattgaataa tgatataata ttcaattaca ttcctgaaag gataaggaat 840
   gacgttaact atatacttaa tatggacaga aatctgccat caacagctag atatataaga 900
   cctaatttac tacaagacag actgaatttg catgacaatt ttgaatcctt gtgggataca 960
   ataacaactt caaactatat tctggcaaga tcggtagtac cagatttaaa ggaattagtt 1020
   tcaaccgaag cgcaaattca aaaaatgtca caagacttgc aactagaagc attaacaata 1080
   cagtcagaaa cgcagttttt aacaggtata aactcacaag cagcaaatga ctgtttcaaa 1140
   actctgattg cagcaatgtt aagtcaacga accatgtcgc ttgatttcgt gactacaaat 1200
   tatatgtcat taatttcagg catgtggtta ctaactgtag tgccaaatga catgttcata 1260
   agggaatcat tggttgcatg tcaactggct atagtgaata caataatata tccagcgttc 1320
   ggaatgcaac gaatgcatta tagaaacgga gacccacaaa caccatttca gatagcagaa 1380
   caacaaatac aaaattttca agtagcgaat tggctgcatt ttgtcaataa caatcaattt 1440
   agacaagtag ttattgatgg tgtattgaat caggtgctga atgacaatat tagaaatgga 1500
   catgtcatta atcaattgat ggaagcttta atgcaactat cacgacaaca gtttccaaca 1560
   atgcctgttg attataagag gtcaatccag cgtggaatat tattgctatc aaataggctt 1620
   ggtcaattag ttgatttaac taggttatta gcttacaact acgaaacact aatggcatgt 1680
   gttacgatga atatgcaaca tgttcagact ttgacaacag aaaaattaca gttaacttca 1740
   gtcacatcgt tgtgtatgct tattggaaat gcaaccgtta tacccagccc gcagacattg 1800
   tttcactatt ataatgttaa tgttaatttt cattcaaatt ataatgaaag aattaatgat 1860
   gcagtggcca taataactgc agctaataga ctaaatttat atcagaaaaa gatgaaggca 1920
   atagttgaag attttttaaa aagattacat attttcgatg tagctagagt tccagatgat 1980
   caaatgtata gattaaggga tagactacga ctattgccag tagaagtaag acgattggat 2040
   atttttaatt tgatactgat gaacatggat cagatagaac gcgcatcaga taaaattgcg 2100
   caaggtgtta ttattgcgta ccgcgatatg caattggaaa gagacgaaat gtatggctac 2160
   gtgaatatag ctagaaattt agatgggttc cagcaaataa acctagaaga attgatgaga 2220
   acaggcgatt atgcacaaat aactaacatg ctcttgaata atcaaccagt agcgctagtt 2280
   ggagctcttc catttgttac agactcgtca gtcatatcgt tgatagcgaa ggttgacgct 2340
   acagtttttg cccaaatagt taaattacgg aaagttgata ccttgaaacc aatattgtat 2400
   aaaataaatt cagattcgaa tgacttttac ctagttgcca actatgattg ggtgcctact 2460
   tcaaccacaa aagtatataa gcaagttcca cagcaatttg atttcagaaa ttcgatgcat 2520
   atgttaacat caaatcttac tttcactgtt tactcagatc tgcttgcatt cgtatcggcc 2580
   gatacagtag aacctataaa tgcagttgca tttgataata tgcgcatcat gaacgagttg 2640
   taa 2643

<210> 2
<211> 2328
<212> DNA
<213> Rotavirus sp.

<400> 2
atggcttcgc tcatttatag acagctgctc actaattcat acacagttga attatctgat 60

gaaattaaaa caattggatc agaaaagagt cagaatgtaa caattaatcc gggtccgttt 120

gctcaaacga cctatgcacc agtcacttgg agacatggag aagtaaacga ttctacaacg 180

gtagaaccag tacttgacgg tccatatcag ccaacgagtt tcaaaccgcc aaatgactat 240

tggatattgt taaacccgat taataaggga gttgtattca agggtactaa caggactgat 300

gtttgggttg caatactact cattgaacaa cgcgtaccta gtcaagatcg acaatataca 360

ttatttggag aagtgaagca aatcactgta gagaatagtt ccgacaaatg gaaattcttt 420
gaaatgttta gaaacaacgc taacattgat tttcagcttc aacgtccttt aacatcagat 480
acaaaattag ctggctttct aacacatggt ggacgtgttt ggacatttaa tggtgaaacg 540
ccgcatgcta caactgatta ctcaacaact tcaaacttac ctgatgtaga agtagtaata 600
catactgaat tctacataat accaagatct caagaatcta aatgcaatga gtatattaat 660
actgggttac caccaatgca aaacacaagg aatgtggttc cagtagcatt atcatctaga 720
tctataactt atcaacgtgc acaagttaac gaagatatca ttatatcaaa gacttcattg 780
tggaaagaaa tgcaatacaa tagagacatt acaataagat ttaaattcgg taatagcata 840
gtaaagcttg gtggattagg ttataaatgg tcagaagtct cattcaaagc agcaaattat 900
cagtataatt atttaaggga tggagaacag gtgacagccc acactacttg ttcagttaac 960
ggagtaaata attttagtta taatggagga tcactgccaa ctgattttag cgtatctaga 1020
tatgaattaa taaaagagaa ttcatatgtt tatatcgatt actgggatga ctcacaagca 1080
ttcaaaaaca tggtatatgt tagatcactt gcagcaaatt taaattcagt gaaatgtagt 1140
ggaggtaact ataactttaa aattccagtt ggtgcatggc cagtaatgag tggtggtgca 1200
gtatctctac atttcgcggg agttacatta tctactcaat ttactaattt cgtatcactc 1260
aattcactaa gattcagatt cagtttaact gttgaggaac catccttttc aattttgcgt 1320
acacgtgtat caggattgta cggattacca gcagctaatc cgaataatgg aaatgaatac 1380
tatgaaatag cgggaagatt ttctctcatt ttattggtac catctaatga cgactatcaa 1440
actccaatta tgaattcagt caccgtacga caagatttag aacgccaatt gggcgatttg 1500
agagaagaat ttaattcact gtcacaagaa atagctatga ctcaattaat agacttggct 1560
ttattgccgt tagatatgtt ttccatgttc tcaggtatta aaagtacaat tgatgtggct 1620
aaatcaatgg ccacaaatgt tatgaaaaag tttaaaaagt caggactagc tacatctata 1680
tcagaactga ctggatcatt gccgagtgct gcatcgtcag tttcaaggag ctcttctatt 1740
agatctaaca tttcatctat ttcagtgtgg acggatgttt ctgaacaaat agcagatgca 1800
tcaaattctg ttagaagtat ttcaacgcag acgtcagcta ttagtaaaag acttagatta 1860
cgtgagatca ctactcagac tgaaggatgg aattttgacg atatttccgc tgctgttctc 1920
aaaacgcccc tagataagtc aacacatata agccctgata cgctgccaga tataataact 1980
gaatcgtctg aaaaatttat accaaaacgc gcttatagag ttttaaagaa tgatgaagtt 2040
atggaggctg atgtagatgg gaaatttttc gcatacagag ttgatacttt cgaagaagtg 2100
ccatttgatg tggataaatt tgttaatctg gccactgctt cccctgtgat atcagctata 2160
attgatttta aaacactgaa aaacctgaat gacaactatg gtataacacg ctctcaagcg 2220
ctagatttga ttagatctga tcccagggtt ctacgtgatt ttatcaatca aaacaatcca 2280
attattaaaa atagaataga acaattaata ctgcaatgta gattgtga 2328

<210> 3
<211> 1194
<212> DNA
<213> Rotavirus sp.

<400> 3
atggatgtcc tgtactcctt gtcaaaaact cttaaagatg ctagagacaa aattgtcgaa 60

ggcacattat actccaatgt aagtgatcta attcaacaat ttaatcaaat gataattact 120

atgaatggaa atgagttcca aactggagga attggtaatc taccgattag aaattggaat 180

tttgattttg gattacttgg aacaactcta ctaaatttag atgctaacta cgtcgaaacg 240

gcccgcaata caattgatta ttttgtagat tttgtagata atgtatgtat ggacgaaatg 300

gttagagaat cacaaagaaa tggaattgca ccacaatcag attcacttat aaagttatca 360

ggcattaaat ttaaaagaat aaattttgac aattcatcag aatacataga gaactggaat 420

ttgccaaata gaagacaaag aacgggtttt acatttcata aaccaaacat tttcccttat 480

tcagcttcat tcacgttgaa cagatcacaa ccggctcatg ataacttgat gggtacgatg 540

tggctcaatg cgggatcaga aattcaggtc gctggattcg actactcatg tgcaataaac 600

gcgccagcta atacgcaaca atttgagcat attgtacagc ttcgaagggt gttgactaca 660

gctacaataa ctcttttacc agatgcagaa agatttagtt ttccaagagt gattacttca 720

gctgacggag cgactacatg gtacttcaat ccagtgattc ttagaccaaa taacgttgaa 780

atagagtttc tactaaacgg gcagataata aatacttacc aagcaagatt tggaacgatc 840

atagctagaa attttgatac aattagattg tcatttcagt tgatgagacc accaaatatg 900

acaccagcgg tagcggcgtt atttccaaat gcgcagccat ttgaacatca cgcaacagta 960

ggactcacgc ttagaattga atctgcagtt tgtgaatcag tacttgccga cgcaagcgaa 1020

acaatgctag caaatgtgac atctgttaga caagaatacg cgataccagt tggaccagtt 1080

tttccaccag gtatgaattg gactgatttg atcactaact attcaccatc tagagaggat 1140

aacttgcagc gtgtatttac agtggcttcc attagaagca tgcttgtcaa atga 1194

<210> 4
<211> 894
<212> DNA
<213> Rotavirus sp.

<400> 4
atggactaca ttatatatag atttttgttg attactgtag cattatttgc tttgacaaga 60

gctcagaatt atggacttaa cttaccaata acaggatcaa tggacgctgt atatactaac 120

tctactcaag aagaagtgtt tctaacttct acgttatgtc tgtattatcc aactgaagca 180

agtactcaaa tcaatgatgg tgactggaaa gactcattgt cgcaaatgtt tcttacaaag 240

ggttggccaa caggatctgt ttactttaaa gagtactcaa atattgttga tttttctgtt 300

gacccacagc tgtattgtga ctataattta gtacttatga aatatgacca aagtcttgaa 360

ttagatatgt cagagttagc tgatttaata ttgaatgaat ggttatgtaa cccaatggat 420

gtaacattat actattatca acaatcggga gaatcaaata agtggatatc gatgggatca 480

tcatgtaccg tgaaagtgtg tccgctaaat acacaaacgt tagggatagg ttgtcaaaca 540

acaaacgtag actcatttga aatgattgct gagaatgaga aattagctat agtggatgtc 600

gttgatggga taaatcataa aataaattta acaactacga catgtactat tcgaaattgt 660

aagaaattag gtccaagaga aaatgtagct gtaatacaag ttggtggttc taatgtgtta 720

gacataacag cagatccaac aactaatcca caaactgaga gaatgatgag agtgaattgg 780

aaaaagtggt ggcaagtatt ttatactata gtagattata ttaatcaaat tgtacaggta 840

atgtccaaaa gatcaagatc attaaattct gcagcttttt attatagagt atag 894

<210> 5
<211> 3105
<212> DNA
<213> Artificial Sequence

<220>
<223> Fusion Protein of rotavirus proteins VP8 and VP2

<400> 5
atggcttcac tcatttatag acagttgctt actaattcat acacagtaga actttcagat 60

gaaatccaag aaattggatc gactaagact caagacgtta ccgttaatcc aggaccgttc 120

gcgcaaacaa attacgctcc agttaattgg ggacctggtg aaacgaatga ctcaactaca 180

gttgaaccag tacttgatgg accatatcaa ccaacgactt ttaatccacc tgtaagttat 240

tggatgttgt tagcaccaac gaacgcgggg gtagtagttg aaggtacgaa caatacaaac 300

agatggttag cgacaatatt aattgaacca aatgtacagc aagttgagcg aacatataca 360

ttatttgggc aacaagttca agtaacagta tcaaataatt cacagacaaa gtggaaattt 420

gtggatctaa gtaagcagac acaagatggt aattattcac aacacggttc tctactgtca 480

acaccgaaac tgtatggagt gatgaaacat ggaggtaaaa tttacactta taatggagag 540

acaccgaacg caactactga ttactactct acaactaact ttgacactgt aaacatgaca 600

gcatattgtg atttttatat aattccatta gcacaagaag caaaatgcac taaatacata 660

aataatggat taccaccaat acaaaatacg agaaatatcg taccagtttc gatagtatca 720

aggtctagag gatccatgaa aacgatacca acatttgaac caaaagagtc aatattgaaa 780

aaattggagg atatcaaacc ggaacaagcg aagaagcaga ctaagctatt tagaatattt 840

gaaccgagac agctaccaat ttatagagcg aatggtgaaa aagagttgcg taacagatgg 900

tattggaagc tgaagaaaga tactttacca gatggagatt atgatgttag agaatacttt 960

ctaaatttgt atgatcaggt tcttactgaa atgccagatt atttactatt aaaagatatg 1020

gcagttgaaa ataaaaattc gagagatgcc ggtaaagttg ttgattctga aacagcaagt 1080

atctgtgatg ctatatttca agatgaggaa acagaaggtg cagtgagacg attcattgcg 1140

gagatgagac agcgcgtaca agctgacaga aacgttgtca attacccatc aatattgcat 1200

ccaatagatt acgcttttaa tgagtatttt ttgcaacacc aattagttga accattgaat 1260

aatgatataa tattcaatta cattcctgaa aggataagga atgacgttaa ctatatactt 1320

aatatggaca gaaatctgcc atcaacagct agatatataa gacctaattt actacaagac 1380

agactgaatt tgcatgacaa ttttgaatcc ttgtgggata caataacaac ttcaaactat 1440

attctggcaa gatcggtagt accagattta aaggaattag tttcaaccga agcgcaaatt 1500

caaaaaatgt cacaagactt gcaactagaa gcattaacaa tacagtcaga aacgcagttt 1560

ttaacaggta taaactcaca agcagcaaat gactgtttca aaactctgat tgcagcaatg 1620

ttaagtcaac gaaccatgtc gcttgatttc gtgactacaa attatatgtc attaatttca 1680

ggcatgtggt tactaactgt agtgccaaat gacatgttca taagggaatc attggttgca 1740

tgtcaactgg ctatagtgaa tacaataata tatccagcgt tcggaatgca acgaatgcat 1800

tatagaaacg gagacccaca aacaccattt cagatagcag aacaacaaat acaaaatttt 1860

caagtagcga attggctgca ttttgtcaat aacaatcaat ttagacaagt agttattgat 1920

ggtgtattga atcaggtgct gaatgacaat attagaaatg gacatgtcat taatcaattg 1980

atggaagctt taatgcaact atcacgacaa cagtttccaa caatgcctgt tgattataag 2040

aggtcaatcc agcgtggaat attattgcta tcaaataggc ttggtcaatt agttgattta 2100

actaggttat tagcttacaa ctacgaaaca ctaatggcat gtgttacgat gaatatgcaa 2160

catgttcaga ctttgacaac agaaaaatta cagttaactt cagtcacatc gttgtgtatg 2220

cttattggaa atgcaaccgt tatacccagc ccgcagacat tgtttcacta ttataatgtt 2280

aatgttaatt ttcattcaaa ttataatgaa agaattaatg atgcagtggc cataataact 2340

gcagctaata gactaaattt atatcagaaa aagatgaagg caatagttga agatttttta 2400

aaaagattac atattttcga tgtagctaga gttccagatg atcaaatgta tagattaagg 2460

gatagactac gactattgcc agtagaagta agacgattgg atatttttaa tttgatactg 2520

atgaacatgg atcagataga acgcgcatca gataaaattg cgcaaggtgt tattattgcg 2580

taccgcgata tgcaattgga aagagacgaa atgtatggct acgtgaatat agctagaaat 2640

ttagatgggt tccagcaaat aaacctagaa gaattgatga gaacaggcga ttatgcacaa 2700

ataactaaca tgctcttgaa taatcaacca gtagcgctag ttggagctct tccatttgtt 2760

acagactcgt cagtcatatc gttgatagcg aaggttgacg ctacagtttt tgcccaaata 2820

gttaaattac ggaaagttga taccttgaaa ccaatattgt ataaaataaa ttcagattcg 2880

aatgactttt acctagttgc caactatgat tgggtgccta cttcaaccac aaaagtatat 2940

aagcaagttc cacagcaatt tgatttcaga aattcgatgc atatgttaac atcaaatctt 3000

actttcactg tttactcaga tctgcttgca ttcgtatcgg ccgatacagt agaacctata 3060

aatgcagttg catttgataa tatgcgcatc atgaacgagt tgtaa 3105

<210> 6
<211> 55
<212> DNA
<213> Artificial Sequence

<220>
<223> oligonucleotide primer

<400> 6
gcggatccac gcgtcattac aggtcctgta tggcgtacag gaaacgtgga gcgcg 55

<210> 7
<211> 23
<212> DNA
<213> Artificial Sequence

<220>
<223> oligonucleotide primer

<400> 7
cacaaggatt caaaattgtc atg 23

<210> 8
<211> 49
<212> DNA
<213> Artificial Sequence

<220>
<223> oligonucleotide primer

<400> 8
aagccaaccc cactgtggct aagcccccaa ttctgcagat atccatcac 49

<210> 9
<211> 22
<212> DNA
<213> Artificial Sequence

<220>
<223> oligonucleotide primer

<400> 9
gcgccgtaca gggcgcgtgg gg 22

<210> 10
<211> 67
<212> DNA
<213> Artificial Sequence

<220>
<223> oligonucleotide primer

<400> 10
ccgccgctaa gcaaaattat tacatatggt ataactaaag aaaatggatg tcctgtactc 60

cttgtca 67

<210> 11
<211> 37
<212> DNA
<213> Artificial Sequence

<220>
<223> oligonucleotide primer

<400> 11
gcgcatttaa atcatttgac aagcatgctt ctaatgg 37


<210> 12
<211> 26
<212> DNA
<213> Artificial Sequence

<220>
<223> oligonucleotide primer

<400> 12
agattttgtc ttcggacacc aactcg 26


<210> 13
<211> 21
<212> DNA
<213> Artificial Sequence

<220>
<223> oligonucleotide primer

<400> 13
tagattgaga gcgtgacctt g 21


<210> 14
<211> 37
<212> DNA
<213> Artificial Sequence

<220>
<223> oligonucleotide primer

<400> 14
gcatttaaat tcatttgaca agcatgcttc taattgg 37


<210> 15
<211> 23
<212> DNA
<213> Artificial Sequence

<220>
<223> oligonucleotide primer

<400> 15
cacaaggatt caaaattgtc atg 23


<210> 16
<211> 534
<212> DNA

<213>  Artificial Sequence

<220>
<223>  Pos. 1-24: TGEV gene 7 TRS inactivated; Pos. 25-261: TGEV gene 7;
       Pos. 262-534  3'UTR

<400>  16
tgatgaggta acgaactaaa cgagatgctc gtcttcctcc atgctgtatt tattacagtt      60

ttaatcttac tactaattgg tagactccaa ttattagaaa gactattact taatcactct     120

ttcaatctta aaactgtcaa tgactttaat atcttatata ggagtttagc agaaaccaga     180

ttactaaaag tggtgcttcg agtaatcttt ctagtcttac taggattttg ctgctacaga     240

ttgttagtca cattaatgta aggcaacccg atgtctaaaa ctggtttttc cgaggaatta     300

ctggtcatcg cgctgtctac tcttgtacag aatggtaagc acgtgtaata ggaggtacaa     360

gcaaccctat tgcatattag gaagtttaga tttgatttgg caatgctaga tttagtaatt     420

tagagaagtt taaagatccg ctacgacgag ccaacaatgg aagagctaac gtctggatct     480

agtgattgtt taaaatgtaa aattgtttga aaattttcct tttgatagtg atac           534


<210>  17
<211>  3768
<212>  DNA
<213>  SARS-CoV

<400>  17
atgtttattt tcttattatt tcttactctc actagtggta gtgaccttga ccggtgcacc      60

acttttgatg atgttcaagc tcctaattac actcaacata cttcatctat gaggggggtt     120

tactatcctg atgaaatttt tagatcagac actctttatt taactcagga tttatttctt     180

ccattttatt ctaatgttac agggtttcat actattaatc atacgtttgg caaccctgtc     240

atacctttta aggatggtat ttattttgct gccacagaga aatcaaatgt tgtccgtggt     300

tgggtttttg gttctaccat gaacaacaag tcacagtcgg tgattattat taacaattct     360

actaatgttg ttatacgagc atgtaacttt gaattgtgtg acaacccttt ctttgctgtt     420

tctaaaccca tgggtacaca gacacatact atgatattcg ataatgcatt taattgcact     480

ttcgagtaca tatctgatgc cttttcgctt gatgtttcag aaaagtcagg taattttaaa     540

cacttacgag agtttgtgtt taaaaataaa gatgggtttc tctatgttta taagggctat     600

caacctatag atgtagttcg tgatctacct tctggtttta acactttgaa acctattttt     660

aagttgcctc ttggtattaa cattacaaat tttagagcca ttcttacagc cttttcacct     720

gctcaagaca tttggggcac gtcagctgca gcctattttg ttggctattt aaagccaact     780

acatttatgc tcaagtatga tgaaaatggt acaatcacag atgctgttga ttgttctcaa     840

aatccacttg ctgaactcaa atgctctgtt aagagctttg agattgacaa aggaatttac     900

cagacctcta atttcagggt tgttccctca ggagatgttg tgagattccc taatattaca     960

aacttgtgtc cttttggaga ggtttttaat gctactaaat tcccttctgt ctatgcatgg    1020

gagagaaaaa aaatttctaa ttgtgttgct gattactctg tgctctacaa ctcaacattt 1080
ttttcaacct ttaagtgcta tggcgtttct gccactaagt tgaatgatct ttgcttctcc 1140
aatgtctatg cagattcttt tgtagtcaag ggagatgatg taagacaaat agcgccagga 1200
caaactggtg ttattgctga ttataattat aaattgccag atgatttcat gggttgtgtc 1260
cttgcttgga atactaggaa cattgatgct acttcaactg gtaattataa ttataaatat 1320
aggtatctta gacatggcaa gcttaggccc tttgagagag acatatctaa tgtgcctttc 1380
tcccctgatg gcaaaccttg caccccacct gctcttaatt gttattggcc attaaatgat 1440
tatggttttt acaccactac tggcattggc taccaacctt acagagttgt agtactttct 1500
tttgaacttt taaatgcacc ggccacggtt tgtggaccaa aattatccac tgaccttatt 1560
aagaaccagt gtgtcaattt taattttaat ggactcactg gtactggtgt gttaactcct 1620
tcttcaaaga gatttcaacc atttcaacaa tttggccgtg atgtttctga tttcactgat 1680
tccgttcgag atcctaaaac atctgaaata ttagacattt caccttgctc ttttgggggt 1740
gtaagtgtaa ttacacctgg aacaaatgct tcatctgaag ttgctgttct atatcaagat 1800
gttaactgca ctgatgtttc tacagcaatt catgcagatc aactcacacc agcttggcgc 1860
atatattcta ctggaaacaa tgtattccag actcaagcag gctgtcttat aggagctgag 1920
catgtcgaca cttcttatga gtgcgacatt cctattggag ctggcatttg tgctagttac 1980
catacagttt ctttattacg tagtactagc caaaaatcta ttgtggctta tactatgtct 2040
ttaggtgctg atagttcaat tgcttactct aataacacca ttgctatacc tactaacttt 2100
tcaattagca ttactacaga agtaatgcct gtttctatgg ctaaaacctc cgtagattgt 2160
aatatgtaca tctgcggaga ttctactgaa tgtgctaatt tgcttctcca atatggtagc 2220
ttttgcacac aactaaatcg tgcactctca ggtattgctg ctgaacagga tcgcaacaca 2280
cgtgaagtgt tcgctcaagt caaacaaatg tacaaaaccc caactttgaa atattttggt 2340
ggttttaatt tttcacaaat attacctgac cctctaaagc caactaagag gtcttttatt 2400
gaggacttgc tctttaataa ggtgacactc gctgatgctg gcttcatgaa gcaatatggc 2460
gaatgcctag gtgatattaa tgctagagat ctcatttgtg cgcagaagtt caatggactt 2520
acagtgttgc cacctctgct cactgatgat atgattgctg cctacactgc tgctctagtt 2580
agtggtactg ccactgctgg atggacattt ggtgctggcg ctgctcttca aatacctttt 2640
gctatgcaaa tggcatatag gttcaatggc attggagtta cccaaaatgt tctctatgag 2700
aaccaaaaac aaatcgccaa ccaatttaac aaggcgatta gtcaaattca agaatcactt 2760
acaacaacat caactgcatt gggcaagctg caagacgttg ttaaccagaa tgctcaagca 2820
ttaaacacac ttgttaaaca acttagctct aattttggtg caatttcaag tgtgctaaat 2880
gatatccttt cgcgacttga taaagtcgag gcggaggtac aaattgacag gttaattaca 2940

ggcagacttc aaagccttca aacctatgta acacaacaac taatcagggc tgctgaaatc 3000

agggcttctg ctaatcttgc tgctactaaa atgtctgagt gtgttcttgg acaatcaaaa 3060

agagttgact tttgtggaaa gggctaccac cttatgtcct tcccacaagc agccccgcat 3120

ggtgttgtct tcctacatgt cacgtatgtg ccatcccagg agaggaactt caccacagcg 3180

ccagcaattt gtcatgaagg caaagcatac ttccctcgtg aaggtgtttt tgtgtttaat 3240

ggcacttctt ggtttattac acagaggaac ttcttttctc cacaaataat tactacagac 3300

aatacatttg tctcaggaaa ttgtgatgtc gttattggca tcattaacaa cacagtttat 3360

gatcctctgc aacctgagct cgactcattc aaagaagagc tggacaagta cttcaaaaat 3420

catacatcac cagatgttga tcttggcgac atttcaggca ttaacgcttc tgtcgtcaac 3480

attcaaaaag aaattgaccg cctcaatgag gtcgctaaaa atttaaatga atcactcatt 3540

gaccttcaag aattgggaaa atatgagcaa tatattaaat ggccttggta tgtttggctc 3600

ggcttcattg ctggactaat tgccatcgtc atggttacaa tcttgctttg ttgcatgact 3660

agttgttgca gttgcctcaa gggtgcatgc tcttgtggtt cttgctgcaa gtttgatgag 3720

gatgactctg agccagttct caagggtgtc aaattacatt acacataa 3768

<210> 18
<211> 666
<212> DNA
<213> SARS-CoV

<400> 18
atggcagaca acggtactat taccgttgag gagcttaaac aactcctgga acaatggaac 60

ctagtaatag gtttcctatt cctagcctgg attatgttac tacaatttgc ctattctaat 120

cggaacaggt ttttgtacat aataaagctt gttttcctct ggctcttgtg gccagtaaca 180

cttgcttgtt ttgtgcttgc tgctgtctac agaattaatt gggtgactgg cgggattgcg 240

attgcaatgg cttgtattgt aggcttgatg tggcttagct acttcgttgc ttccttcagg 300

ctgtttgctc gtacccgctc aatgtggtca ttcaacccag aaacaaacat tcttctcaat 360

gtgcctctcc gggggacaat tgtgaccaga ccgctcatgg aaagtgaact tgtcattggt 420

gctgtgatca ttcgtggtca cttgcgaatg gccggacacc ccctagggcg ctgtgacatt 480

aaggacctgc caaaagagat cactgtggct acatcacgaa cgctttctta ttacaaatta 540

ggagcgtcgc agcgtgtagg cactgattca ggttttgctg catacaaccg ctaccgtatt 600

ggaaactata aattaaatac agaccacgcc ggtagcaacg acaatattgc tttgctagta 660

cagtaa 666

<210> 19
<211> 231
<212> DNA
<213> SARS-CoV

<400>  19
atgtactcat tcgtttcgga agaaacaggt acgttaatag ttaatagcgt acttcttttt      60

cttgctttcg tggtattctt gctagtcaca ctagccatcc ttactgcgct tcgattgtgt     120

gcgtactgct gcaatattgt taacgtgagt ttagtaaaac caacggttta cgtctactcg     180

cgtgttaaaa atctgaactc ttctgaagga gttcctgatc ttctggtcta a              231

## Description

The present invention is directed to nucleic acids comprising sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell. The nucleic acids of the present invention further encode one or more proteins of a different virus, which is not FMDV, wherein these one or more proteins are capable of associating into a virus-like particle (VLP) that does not contain any infectious nucleic acid. The present invention is further directed to the use of these nucleic acids for the preparation of pharmaceutical compositions in general and specifically for the preparation of vaccines.

### TECHNICAL BACKRGOUND

Therapy approaches that involve the insertion of a functional gene into a cell to achieve a therapeutic effect are also referred to as gene therapy approaches, as the gene serves as a drug. Gene therapy is a technique primarily for correcting defective genes responsible for disease development.

A carrier molecule also referred to as a vector is used to deliver the therapeutic gene to the patient's target cells. Currently, the most common vector is a virus that has been genetically altered to carry human or animal genes. Viruses have evolved a way of encapsulating and delivering their genes to human or animal cells in a pathogenic manner. Scientists have taken advantage of this capability and manipulate the virus genome to remove disease-causing genes and insert therapeutic genes.

Target cells such as the patient's liver or lung cells are infected with the viral vector. The vector then unloads its genetic material containing the therapeutic gene into the target cell. The generation of a functional protein product from the therapeutic gene restores the target cell to a normal state.

In an alternative approach, these viral vectors were used for expressing heterologous genes that cause an immunogenic response in the subject receiving the vector and thus immunize that subject. In that case the viral vector serves as a vaccine.

Transmissible gastroenteritis virus is a member of the family of coronaviruses. Coronaviruses are ssRNA(+) viruses which have the largest genome so far found in RNA viruses with a length between 25 and 31 kilobases (kb; see Siddell S.G. 1995, The Coronaviridae). When a coronavirus infects a cell, the genomic RNA (gRNA) replicates in the cytoplasm and a set of subgenomic RNAs (sgRNA) of positive and negative polarity is produced (Sethna et al., 1989; Sawicki & Sawicki, J.Virol., 1990; and Van der Most and Spaan, The Coronaviridae).

Due to the fact that the coronaviruses replicate in the cytoplasm, use of coronaviruses as a vector for gene therapy and vaccination has been suggested (Enjuanes et al., 2003). Specifically, defective interfering (DI) genomes of coronaviruses were produced. These DI genomes are deletion mutants which require the presence of a complementing or helper virus for replication and/or transcription (see Chang et al., 1994; WO97/34008; Spanish patent application P9600620; Izeta et al., 1999; and Sánchez et al., 1999).

A respective system was used in the art to generate immune responses in an animal which received a composition containing a DI genome which amongst others contained sequences encoding a heterologous reporter gene or a gene derived from a different infectious agent (porcine reproductive and respiratory disease virus, PRRSV; see Alonso et al., 2002a, 2002b).

The entire genome of a coronavirus was cloned in the form of an infectious cDNA (Almazan et al., 2000 and WO01/39797). The cloning of the entire genome allowed the preparation of infectious vectors containing heterologous sequences suitable for expression of large proteins in a host cell. In the examples of WO01/39797 sequences encoding the ORF 5 of the porcine reproductive and respiratory disease virus (PRRSV) were expressed in a viral vector derived from a coronavirus.

The potential of the cloned viral genome for expression of heterologous sequences was further reviewed in Enjuanes et al., 2003.

Using the cloned virus the structure of the genome and relevance of the coronaviral genes for infection were assessed by preparing deletion mutants. It was found that genes 3a, 3b and 7 are non-essential for replication of the viral nucleic acid and that absence of the genes reduces pathogenicity of the virus (Ortego et al., 2002 and 2003; Sola et al., 2003).

The preparation of a vaccine requires that the viral sequences are expressed in a manner which closely resembles the virus particle during infection. Therefore it was suggested to prepare virus-like particles, i.e. an association of several proteins of one virus that resembles the virus. The virus-like particles (VLPs) were administered as a vaccine for example intranasally (see Schwartz-Cornil et al., 2002). Kim et al. (2002), for example describe the production of rotavirus virus-like particles consisting of bovine VP6 and VP2 proteins by expressing the genes encoding these proteins in recombinant baculoviruses.

The rotaviruses, members of the family reoviridae, are the most important agent causing of severe viral gastroenteritis in humans and animals. Morphologically, the capsid consists of three concentric layers. The outermost layer in the infectious virus is composed of the glycoprotein VP7 and the spike protein VP4 that is the virus attachment protein. Following a rotavirus infection, a humoral immune response is elicited that comprises the production of antibodies against VP7 and VP4 that induce a protective immunity.

The intermediate capsid layer is composed of trimers of VP6 organized on T=13 icosahedral lattice. The innermost capsid layer is composed of 120 molecules of a 102-kDa protein (VP2) and encloses the genomic dsRNA. Rotavirus genome consists of 11 segments of base paired double stranded RNA with a size range from 0.6 to 3.3 Kbp.

VP4 is an unglycosylated protein of the rotavirus outer layer, present on the surface of the outer layer of the mature virions as 60 dimer spikes with lobed heads. VP4 induces neutralising antibodies and protective immunity in animals and in children. Efficient infectivity of rotavirus in cell culture requires trypsin cleavage of VP4 into fragments VP8* (28 KDa) and VP5* (60 KDa), respectively the N- and C-terminal portion of VP4. VP8* is the viral hemagglutinin and is an activator of intracellular signalling pathways.

All of the above approaches are directed to the use of viral proteins as a vaccine. In a different approach WO02/092827 suggested a vaccine comprising coronavirus virus-like particles from a coronavirus vector backbone. Large parts of the coronaviral genome had been (at least functionally) deleted. However, in that publication virus-like particles were defined as particles containing proteins and nucleic acid (viral RNA). The virus like particle thus contains a potentially replicative nucleic acid and is thus much less safe than the VLPs administered directly as a protein vaccine.

The problem underlying the present invention thus resides in providing vaccine vectors with good safety and immunogenicity.

### SUMMARY OF THE INVENTION

According to a first aspect of the present invention a nucleic acid is provided which comprises:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of a different virus, which is not FMDV, wherein these one or more proteins are capable of associating into a virus-like particle (VLP) that does not contain any infectious nucleic acid.

The replication competent TGEV sequences need not but may further encode other TGEV proteins. The TGEV sequences may thus encode a fully infectious TGEV virus and the sequences of the further virus or just comprise a replication competent nucleic acid. The present invention further relates to vectors comprising a respective nucleic acid and host cells comprising the vector. The host cells may be capable of complementing TGEV genes that may have been deleted from the nucleic acids of the present invention. The host cell thus may be a packaging cell line or may contain a helper virus expressing TGEV genes, so that a TGEV virus particle is formed that comprises the sequences of a different virus, which is not FMDV, encoding proteins capable of associating into a virus-like particle (VLP) that does not contain any infectious nucleic acid. Virus particles obtained by association of the TGEV coat proteins with the replication competent but non-infectious nucleic acids of the present invention are an especially preferred embodiment of the present invention (corresponding virus particles have also been referred to as pseudoviruses).
Finally, the present invention is also directed to the medical use of the nucleic acids, the virus vectors, the host cells and the virus particles, specifically to the use as a vaccine for treating or protecting animals, such as a human, a ruminant or a swine against infectious diseases. The vaccine can thus be administered to a human or an animal to reduce or eliminate the symptoms of a subsequent infection of a wild-type virus.

### DETAILED DESCRIPTION OF THE INVENTION

The present invention is thus directed to a nucleic acid comprising:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of a different virus, which is not FMDV, wherein these one or more proteins are capable of associating into a virus-like particle (VLP) that does not contain any infectious nucleic acid.

The present inventors have surprisingly found that expression of heterologous proteins in the context of the TGEV vector backbone allows the foreign proteins to associate into a virus-like particle, when the nucleic acid is expressed in a host cell.

In accordance with the present invention an association of viral proteins is referred to as a "virus-like particle" if it comprises a covalently coupled or otherwise linked association of one viral protein with at least a part of a further viral protein that may have the same or a different sequence. Preferably the particle comprises an association of at least two or three or all of the different viral coat proteins. The "virus-like particle" does not contain any replicating nucleic acid and is by itself thus not capable of causing an infection.

In other words, the nucleic acids of the present invention allow expression and secretion of virus-like particles, which will initiate immune responses closely mimicking the immune response caused by the wild-type virus particle. At the same time the virus-like particles do not contain any infectious nucleic acids, i.e. are extremely safe and cannot per se cause any infection.

Although the VLPs thus do not contain a nucleic acid, the proteins of the VLPs are encoded by a nucleic acid of the present invention. In its broadest aspect that nucleic acid is further characterized as a nucleic acid encoding replication competent TGEV sequences that means sequences encoding a TGEV replicase under the control of expression regulatory sequences so that expression of the replicase in a cell containing the nucleic acid will initiate replication of the nucleic acid and thus increase the number of nucleic acids in the cell. Once a cell is infected by the nucleic acids of the present invention, the gene for the replicase will be expressed and the nucleic acid will be replicated. The more copies of the nucleic acid are present in the cell the more VLPs will be expressed.

The replication competent TGEV vector may be infectious or not. A nucleic acid that contains at least all sequences necessary for replication of the nucleic acid, produces one or several coat proteins and associates with the coat proteins to a viral particle that will allow infection of other cells is referred to as an infectious nucleic acid in accordance with the present invention.

The non-infectious vector is more safe, as it does not contain sequences encoding a protein capable of associating with the nucleic acid to form an infectious viral particle.

However, the infectious TGEV vector also has advantages, as it will spread in the vaccinated animal and thus increase the immune response against the VLPs. According to one aspect of the present invention, the TGEV vector encodes different coat proteins that will allow infection of different host cells of the same organism.

In an especially preferred aspect, the present invention provides a virus particle that comprises the above nucleic acid and at least one TGEV coat protein. The virus particle may comprise more than one and even all TGEV coat proteins. A corresponding virus particle will be capable of entering a host cell by way of infection. However, the nucleic acid of such a virus particle may still be infectious or non-infectious, as it need not encode all of the TGEV coat proteins necessary to produce a virus particle. If the nucleic acid is a non-infectious nucleic acid in the sense of the present application, the virus particle is prepared using a packaging host cell or a helper virus that complements the TGEV genes. The use of packaging host cells or helper viruses for obtaining virus particles comprising an incomplete genome of a virus is well known in the art. This way of proceeding has specific advantages, as the virus particle is per se infectious (i.e. can infect a cell once) but the nucleic acid is not capable of producing further infectious virus particles. In other words, neither the sequences derived from TGEV nor the sequences derived from the different virus encode proteins that will be capable of associating with the nucleic acid to form a new virus particle. These virus particles thus are extremely safe and still provide a high immunogenic response against the VLPs expressed by the nucleic acids.

According to an alternative embodiment of the present invention the TGEV infectious viral particles obtainable from the association of TGEV proteins and the nucleic acid sequences are attenuated viral particles. This has the advantage that the subject to be treated using the nucleic acids of the present invention will be vaccinated at the same time against TGEV and against the virus corresponding to the VLPs.

The nucleic acids of the present invention may comprise sequences encoding all proteins of TGEV. Alternatively, the nucleic acids may comprise sequences only encoding the TGEV proteins needed for a replication compentent TGEV vector. The nucleic thus preferably encodes the TGEV replicase. According to an especially preferred embodiment, the nucleic acid encodes a replication competent but non-infectious TGEV vector that comprises sequences encoding the TGEV replicase and the TGEV N protein and none of the other TGEV proteins. This vector has the specific advantage that the TGEV vector will be highly amplified in the host cell and thus produce large amounts of the VLPs. At the same time this vector is extremely safe as it is non-infectious.

The term "nucleic acids encoding TGEV proteins" is used herein to refer to nucleic acid sequences as disclosed in Penzes et al., 2001 or nucleic acid sequences having a homology of at least 60%, preferably at least 75% and most preferably at least 95% to these sequences. For example specific alternative sequences may be used to differentiate between TGEV vaccinated animals and TGEV infected animals (as outlined in more detail below). In the TGEV based vector exemplified in the present application corresponding nucleotide substitutions have been introduced using RT-PCR at positions 6752, 18997, 20460, and 21369, respectively. Especially nucleic acid sequences encoding the TGEV replicase, N protein, M protein, E protein or S protein of TGEV as used herein means nucleic acid sequences as disclosed in Penzes et al., 2001 (with or without the amendments mentioned above). It is of course also possible to use other TGEV strains or to include further deletions substitutions, insertions or additions into the nucleic acid sequence. According to a further aspect the TGEV sequences thus differ from the sequences disclosed in Penzes et al. but still have a homology of at least 60%, preferably at least 75% and most preferably at least 95% to these sequences.

For the purposes of the present application sequence homology is determined using the clustal computer program available from the European Bioinformatics Institute (EBI), unless otherwise stated.

The nucleic acid of the present invention may further encode SEQ ID NO: 16 (gene 7 TRS inactivated, plus the UTR 3'primer).

The infectious TGEV vector need not contain genes 3a, 3b and 7, as these are known to be non-essential. The proteins encoded by genes 3a, 3b and 7 of TGEV may modulate the immune response against TGEV and where it is desirable to modulate TGEV interaction with the host, these genes may be maintained in the TGEV vector.

The protein coding sequences within the nucleic acids of the present invention are preferably linked to sequences controlling the expression of these genes in the host cells or organisms. The genes encoding proteins capable of associating into VLPs may for example be flanked by transcription regulatory sequences (TRS) and/or internal ribosome entry site (IRES) sequences to increase transcription and/or translation of the protein coding sequences. Respective TRS and IRES sequences are well known in the art.

The proteins that are capable of associating into VLPs can be expressed as a polyprotein or can be encoded by seperate genes. When expressed as a polyprotein, the nucleic acid of the present invention preferably also encode a protease that is capable of digesting the polyprotein into sperate proteins capable of associating into VLPs.

According one alternative of the present invention, the nucleic acids encoding proteins of a non-TGEV virus are immunogenic proteins of a rotavirus. The rotavirus virus-like particles thus produced are capable of generating an immune response in a mammal. The nucleic acids of the present invention may comprise sequences encoding at least two of rotavirus proteins capable of associating into a virus like particle, such as VP2 (SEQ ID NO:1), VP4 (SEQ ID NO:2), VP6 (SEQ ID NO:3) or VP7 (SEQ ID NO:4) or sequences having a homology of at least 60%, preferably at least 75% and most preferably at least 95% to the SEQ ID NO: 1 to 4.

In a preferred embodiment of the invention the nucleic acids comprise sequences encoding rotavirus proteins VP2 (SEQ ID NO:1) and VP6 (SEQ ID NO:3) or sequences having a homology of at least 60%, preferably at least 75% and most preferably at least 95% to SEQ ID NO:1 or 3.

Furthermore, the nucleic acids of the present invention may comprise sequences encoding fusion proteins comprising sequences derived from rotavirus proteins. A nucleic acid of the present invention may thus comprise sequences encoding a fusion protein comprising rotavirus proteins VP8 and VP2 (SEQ ID NO:5) or sequences having a homology of at least 60%, preferably at least 75% and most preferably at least 95% to the SEQ ID NO:5.

The genes encoding rotavirus VLPs may be derived from the same or from a different species, i.e. may be derived from a human or animal rotavirus , e.g. from a human and/or bovine rotavirus.

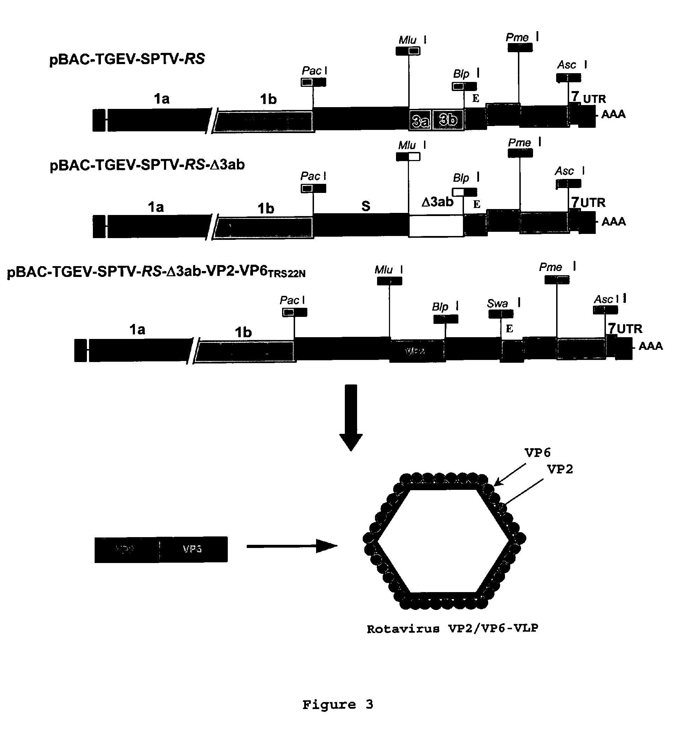

In one embodiment , the non-essential genes ORFs 3a and 3b are eliminated from the full-length cDNA clone, creating a deletion in the TGEV genome and the heterologous genes ORF2 and ORF6 that encodes rotavirus structural proteins VP2 and VP6 are inserted in the cDNA construct, replacing the deleted TGEV ORFs 3a and 3b (Figure 3). The resultant cDNA encodes the recombinant virus rTGEV-S_{PTV}-*RS*-Δ3ab-VP2-VP6_{TRS22N} encoding the rotavirus ORF2 gene under the transcription-regulatory sequences (TRS) of ORF 3a; and the ORF6 gene under the engineered TRS including the 5'TRS from N gene (TRS_{N}) that was inserted just downstream of the rotavirus ORF2 gene stop codon. The recombinant viral vector encodes rotavirus structural proteins VP2 and VP6 and stably directs the expression of high levels of rotavirus virus-like particles. TGEV virus particles comprising the above recombinant viral vector, rTGEV-S_{PTV}-*RS*-Δ3ab-VP2-VP6_{TRS22N}, encapsulated by TGEV coat proteins were deposited according to the provisions of the Budapest Treaty with the Institute Pasteur (Paris, France) on August 31, 2004 under the Registration Number CNCM I-3289.

According to a further embodiment the nucleic acids of the present invention comprise sequences derived from the Severe Acute Respiratory Syndrome (SARS) virus which sequences are capable of associating into a virus like particle. SARS is a coronavirus causing severe pneumonia in humans that will lead to death in a number of infected patients. The present invention provides a nucleic acid comprising genes encoding SARS proteins capable of associating into a virus like particle which nucleic acids can be used to prepare a safe SARS vaccine. While the nucleic acids may encode any SARS protein capable of associating into a VLP, they preferably encode SARS M (SEQ ID NO:18), E (SEQ ID NO:19) and S (SEQ ID NO:17) genes or sequences having a homology of at least 60%, preferably at least 75% or at least 95% to the sequences identified. Upon expression of this nucleic acid in a host cell the same will produce VPLs comprising SARS proteins S, M and E.

According to a further alternative embodiment, the nucleic acids of the present invention comprise sequences derived from porcine circovirus (PCV) which sequences are capable of associating into a virus like particle. Again, the nucleic acids may encode any PCV protein capable of associating into a VLP, but preferably encodes ORF2 of PCV and upon expression of the nucleic acid in a host cell produces VPLs comprising PCV proteins encoded by ORF2.

According to a further alternative embodiment, the nucleic acids of the present invention comprise sequences derived from parvovirus which sequences are capable of associating into a virus like particle. The nucleic acids may encode any parvovirus protein capable of associating into a VLP, but preferably encodes parvovirus capsid protein gene and upon expression of the nucleic acid in a host cell produces VPLs of the parvovirus capsid protein.

The nucleic acids of the present invention may be in the form of DNA or RNA. Within the scope of the present invention specifically recombinant RNA molecules are encompassed which are encoded by one of the above nucleic acids.

According to a further aspect the present invention is directed towards vectors comprising one of the above nucleic acids. The vector can be a cDNA vector and preferably is a BAC derived vector, such as BAC-TGEV^{FL}. The vector is preferably capable of replicating the nucleic acid within a specific host cell or a number of host cells.

Host cells, which comprise a vector comprising one of the above nucleic acids are a further subject of the present invention. The cell may be a bacterial cell, a yeast cell, an insect cell, an animal cell or a human cell. According to a preferred embodiment the cell is a porcine swine testis cell line, such as the cell line deposited under ATCC CRL1746.

A further aspect of the present invention is directed to methods of preparing virus-like particles that do not contain any infectious nucleic acid which methods comprise steps, wherein a nucleic acid sequence as described above is expressed in a host cell in cell culture and the virus-like particles are isolated from the medium and/or from the host cells.

The present invention further is directed to pharmaceutical compositions comprising one of the nucleic acids, viral RNAs or vectors of the present invention or a host cell as described above. The pharmaceutical composition may further comprise a pharmaceutically acceptable carrier, excipient and/or adjuvants.

In a further embodiment the present invention relates to vaccines capable of protecting an animal against the disease caused by an infectious virus comprising a nucleic acid, a viral RNA, a vector or a host cell of the present invention. The vaccine may also comprise pharmaceutically acceptable carriers, excipients and/or adjuvants.

Adjuvants and carriers suitable for administering genetic vaccines and immunogens via the mucosal route are known in the art. Conventional carriers and adjuvants are for example reviewed in Kiyono et al 1996. The addition of chemokines that are used to modulate immune responses are also encompassed by the present invention. Respective compounds and their medical use has been reviewed in Toka et al. 2004. It is specifically advantagous to use one of granulocyte/macrophage colony-stimulating factor, interleukin-2 (IL-2), IL-12, IL-18. Combinatorial approaches utilizing several cytokines and chemokines might also be applied. In addition, as more is discovered regarding the requirements for memory development of T cells, boosters involving key cytokines such as IL-15 and IL-23 may prove beneficial to long-term maintenance of the memory pool.

The vaccine is preferably suitable for treating a mammal, for example a ruminant or a swine.

In accordance with the present invention vaccines are provided, which are preferably capable of inducing both a systemic immune response and a mucosal immune response against infectious viral agents, such as rotavirus, SARS, PCV, parvovirus and/or TGEV.

The vaccine may be administered in accordance with methods routinely used in the art. Specifically vaccine may be administered by intramuscular, intravenous or oronasal administration.

The vaccines of the present invention allow one of ordinary skill to diagnose whether an animal is infected with a wild-type virus or has been vaccinated. According to a further aspect, the present invention is thus directed to methods for diagnosing whether an animal is infected with a virus or has been vaccinated using a vaccine of the present invention, which methods comprise steps, wherein the diagnosis uses antibodies specific for proteins of the wildtype virus not expressed by the vaccine strain. Differentiation of TGEV vaccinated animals from TGEV infected animals could alternatively be carried out using RT-PCR and sequence markers introduced into the recombinant TGEV genome at positions 6752, 18997, 20460, and 21369, which should encode G, C, T, and C, respectively.

The differentiation between vaccinated animals and wildtype rotavirus or PCV infected animals can be carried out using antibodies specific for proteins not present in the recombinant virus.

### Brief description of the Figures:

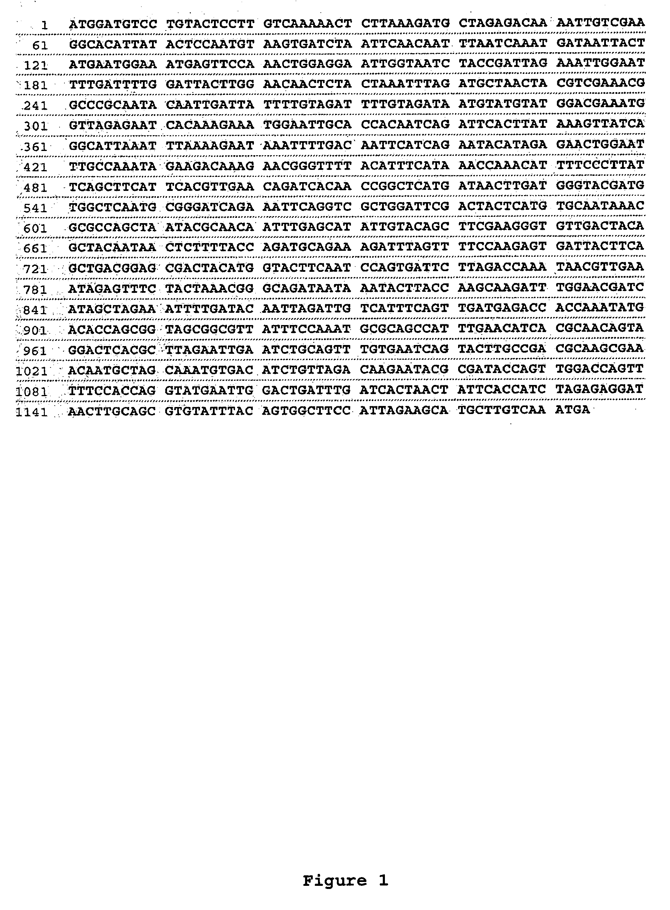

Figure 1 shows the nucleic acid sequence (SEQ ID NO:3) corresponding to rotavirus ORF6 that encodes the rotavirus structural protein VP6.

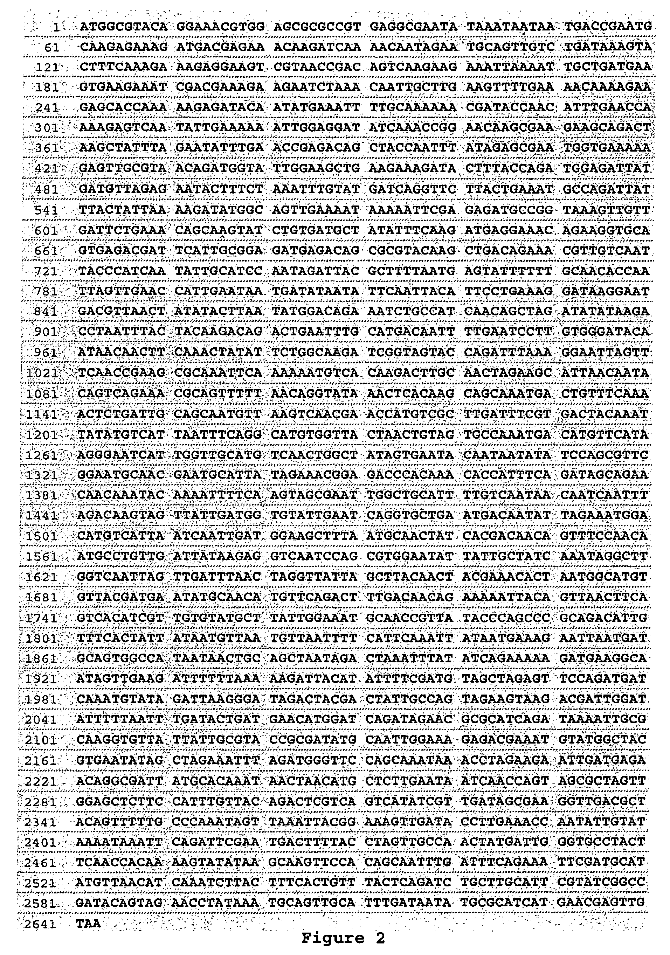

Figure 2 shows the nucleic acid sequence (SEQ ID NO:1) corresponding to rotavirus ORF2 that encodes the rotavirus protein VP2.
Figure 3 shows the schematic structure of the cDNA encoding the rTGEV-S_{PTV}-*RS*-Δ3ab-VP2-VP6_{TRS22N} . As shown the heterologous rotavirus genes ORF2 and ORF6 were cloned in the same rTGEV viral vector. The heterologous rotavirus ORF 2 gene was cloned under the TRS of the TGEV ORF 3a while the ORF6 gene under the engineered TRS_{22N}. The arrangement of rotavirus VP2/VP6-VLPs is pictured below. Numbers, letters indicate the viral and heterologous rotavirus genes. Unique restriction sites *(RS)* are shown in italics.

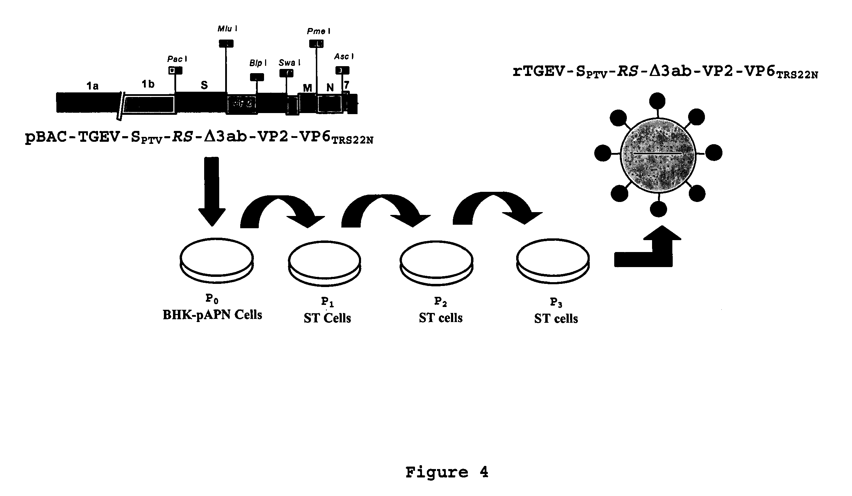

In Figure 4 the experimental procedure used in the recovery and amplification of rTGEV-S_{PTV}-*RS*-Δ3ab-VP2-VP6_{TRS22N} is displayed. As first step the BHK-pAPN cells were transfected with the plasmid pBAC-TGEV-S_{PTV}-*RS*-Δ3ab-VP2-VP6_{TRS22N}. For amplification, the recombinant virus generated was passaged three consecutive times into confluent ST cells (P1, P2 and P3).
Figure 5 shows ST cells infected with the rTGEV-S_{PTV}-*RS*-Δ3ab-VP2-VP6_{TRS22N} and the analysis of the VP2 and VP6 structural proteins expression by confocal microscopy.
Figure 6 shows the assembly of rotavirus VP2/VP6-VLPs in ST cells infected with the rTGEV-S_{PTV}-*RS*-Δ3ab-VP2-VP6_{TRS22N} by electron microscopy.
In Figure 7 the detection of the mRNA of the TGEV N gene and Rotavirus ORF2 / ORF6 genes in ST cells infected with the rTGEV-S_{PTV}-*RS*-Δ3ab-VP2-VP6_{TRS22N} is shown. mRNA was detected at different passages (P1-P3) by RT-PCR assay. The reaction products were visualized by agarose gel electrophoresis.
Figure 8 shows the Western blot detection of VP2 and VP6 proteins expressed by the rTGEV-S_{PTV}-*RS*-Δ3ab-VP2-VP6_{TRS22N} in the lysates of infected swine testis cells (ST). Cell lysates from passage 3 were separated by SDS-PAGE under reducing conditions and probed with monoclonal antibodies specific for TGEV N protein or rotavirus VP2 and VP6 structural proteins.

The following examples illustrate the preparation of virus-like-particles according to the invention.

### EXAMPLE 1

### Growth of Eukaryotic Cells

TGEV growth, titration, and purification were performed in ST (swine testicle) cells, a cell line obtained from epithelial cells of fetal pig testicles (McClurkin and Norman, 1966). ST cells were obtained from L. Kemeny (National Animal Disease Centre, Ames, Iowa, USA).

Plasmid transfections assays were performed in Baby Hamster Kidney cells (BHK-21) stably transformed with the gene coding for the porcine aminopeptidase N (BHK-pAPN) (Laude et al., 1990). ST cells were cultivated in DMEM (Dulbecco's Modified Eagle Medium) supplemented with 10% fetal calf serum (FCS) (GIBCO-BRL), 50 mg/mL gentamicine, 2 mM glutamine, and 1% non-essential amino acids.

The BHK-21 stably transformed with the gene encoding for the porcine aminopeptidase N (BHK-pAPN) were grown in DMEM (Dulbecco's Modified Eagle Medium) supplemented with 2% fetal calf serum (FCS) (GIBCO-BRL), 50 mg/mL gentamicine, 2 mM glutamine, and 1% non-essential amino acids and Geneticine (G418) (1,5 mg/ml) as a selection agent.

### EXAMPLE 2

### Transformation of bacteria by plasmid electroporation

### Bacterial strains:

*Escherichia coli* DH10B (Gibco/BRL) (Hanahan et al., 1991) was the host for all the plasmids constructed. The genotype of this bacterial strain is: F⁻mcr A Δ (mrr-hsdRMS-mcrBC) φ80d*lac*ZΔM15 Δ*lac*X74 *deo*R *rec*A1 *end*A1 *ara*D139 (*ara, leu*) 7697 *gal*U *gal*K_*rsp*L *nup*G.

### Preparation of competent bacteria:

For amplification and production of electroporation-competent *E. coli* DH10B bacteria, the bacteria were grown in a SOB medium. Were inoculated 10 mL of SOB medium (20 g/L tryptone, 5 g/L yeast extract, 0,5 g/L NaCl) with a colony from a fresh plate, and were incubated for 12 h at 37°C under agitation. With 2 mL of this culture, 1 L of SOB medium supplemented was inoculated, and the culture was grown at 37°C to an optical density of 600 nm, between 0.8 and 0.9 absorbance units. Then the culture was cooled on ice for 20 min, and the bacteria were centrifuged in the Sorvall GSA rotor at 4,000 G for 15 min at 4°C. The bacteria were resuspended cold in 1 L of 10% glycerol. The bacteria suspension was centrifuged again and resuspended in 500 mL of 10% cold glycerol. The bacteria were sedimented and resuspended in 250 mL of 10% cold glycerol. Finally, the bacteria were centrifuged and resuspended in 3 mL of 10% glycerol. The final suspension was divided into aliquots parts of 50 µL and 100 µL and were kept at -70°C until they were used for electroporation. The transformation efficiency of the bacteria was calculated by electroporation with a known concentration of a pBluescript plasmid as a reference, and was reproducibly at about 10⁹ colonies/µg of DNA.

### Transformation of bacteria by plasmid electroporation:

50 µL of transformation-competent bacteria were mixed with 1 µL of each reaction mixture, or 10 ng of purified plasmid to the bacteria and incubated on ice for 1 min. Then, the mixture was transferred to 0.2 cm electroporation trays (Bio-Rad), and were transformed by a 2.5 kV electric pulse, 25 µF and 200 Ω in a "Gene Pulser" electroporator (Bio-Rad). After adding 1 mL of cold LB medium, the bacteria were incubated at 37°C under agitation for 1 h. Between 50 and 100 µL of the suspension of transformed bacteria were seeded in Petri dishes with LB (Luria-Bertani medium) in a solid medium (15 g/L agar) supplemented with ampicillin (100 µg/mL) or chloramphenicol (34 µg/mL). The bacteria grew for 16 h at 37°C (Bulllock, et al. 1987).

For production and purification of plasmids, the bacteria transformed with plasmids, that conferred ampicillin or chloramphenicol resistance, were grown from an isolated colony on a plate, in a liquid LB medium supplemented with 100 µg/mL of ampicillin or 34 µg/mL of chloramphenicol.

### EXAMPLE 3

### Plasmids for cloning of PCR products

The pGEM-T (Promega) plasmid was used to clone PCR products. This plasmid contains the T7 and SP6 bacteriophage promoters separated by the LacZ gene, interrupted by two protuberant T sequences between a multicloning sequences. This plasmid confers ampicillin resistance for its selection.

### EXAMPLE 4

### Manipulation of DNA

### Cloning and restriction enzymes:

For the manipulation and cloning of DNA, the restriction enzymes *Bam*HI, *Bbs* I, *Blp* I*, Eco* RI, *Mlu* I*, Swa* I, *Xcm* I*, Xho* I, were acquired from ROCHE or from New England Biolabs. Dephosphorylation of the DNA terminals, was done with shrimp alkaline phosphatase (SAP) (USB). A DNA ligation as T4 phage DNA ligase (New England Biolabs) was used. All the treatment with restriction enzymes, dephosphorylation, and DNA ligation were carried out by standard protocols previously described (Sambrook et al., 1989).

### Polymerase chain reaction (PCR):

To amplify DNA from a matrix, frequently plasmids, 50-100 ng of DNA plasmid was mixed with the corresponding oligonucleotides (10 µM), 0.25 mM deoxynucleotides triphosphate (ATP, GTP, TTP, and CTP), 1.25 mM MgCl₂, PCR buffer (10 mM Tris-HCl, pH 8.3, 50 mM KCl) and 2.5 U of Taq Gold DNA polymerase (*Thermus aquaticus*) (Roche), in a final volume of 50 µL. The reactions were carried out in the GeneAmp PCR System 9600 thermocycler from Perkin Elmer.

### Separation of DNA by agarose gel electrophoresis:

To separate DNA fragments, 1% agarose gels were used with ethydium bromide (1 µg/mL) in a 1X TAE buffer (40 mM Tris-acetate, 1mM EDTA).

### Purification of DNA:

The bacterial plasmids grown in the presence of the selection antibiotics were purified using the "Qiaprep Spin Miniprep kit" (Qiagen) to prepare of small quantities plasmid DNA, and the "Qiafilter Midi-Plasmid Kit" system (Qiagen) to prepare intermediate quantities of plasmid DNA. The DNA obtained from agarose gels was purified using the "QiaEx II Gel Extraction Kit" system (Qiagen). Purification of the PCR products was carried out by means of the system "QIA quick PCR Purification Kit" (Qiagen). In all cases, the manufacturer's instructions were followed.

### EXAMPLE 5

### RNA analysis

For analysis of the RNA produced in infections with TGEV clone PUR46-MAD, confluent monolayers of ST cells grown in 60 mm diameter culture plates (NUNC) were infected with viral inocula at a MOI of 1. The cells were lysed at 16 hpi [hours post infection] using a "RNeasy Mini Kit" (Qiagen) , following the protocol provided by the commercial firm (Qiagen). The RNA was purified and resuspended in 40 µL of water treated with DEPC [diethyl polycarbonate] and 20 U of RNAse inhibitor (Roche).

### EXAMPLE 6

### Transfection and Recovery of infectious TGEV from cDNAs Clones

BHK-pAPN cells (Delmas, 1994) were grown in Dulbecco's modified Eagle's medium (DMEM) supplemented with 2% fetal calf serum and containing Geneticin (G418) (1,5 mg/ml) as a selection agent. BHK-pAPN cells were grown to 60% confluence in 35-mm-diameter plates and transfected with 10µg of pBAC-S_{PTV}-*RS*-Δ3ab-VP2-VP6_{TRS22N} plasmid with 15µl of lipofectin (GIBCO Life Technologies) according to the manufacturer's specifications. The cells were incubated at 37°C 5% CO₂ and after 6 h the transfection medium was replaced with fresh DMEM containing 5% (vol/vol) FBS. Two days later (referred to as passage 0), the cells supernatants were harvested and passaged four times on fresh ST monolayer to increase rTGEV titer. Virus titers were determined by plaque titration.

Alternatively, ST cells were grown in 25 cm² culture flask using DMEM (Dubelco's Minimal Essential Medium) 10% SFB (Serum Fetal Bovine) at 90% of confluence and infected at a MOI (multiplicity of infection) of 1 plaque unit formation per cell. The supernatant was recovered after 48 hours and titrated by plaque limit dilution.

### Plaque titration:

Titration of viral stocks was made by plaque limit dilution assay on 24-well cultured ST cells to quantify the number of infective particles. ST cells were grown in 24-multiwell culture plate at 90 % of confluence. Recombinant TGEV viruses were 10-fold serially diluted (10E-1, 10E-2, 10E-3, 10E-4, 10E-5, 10E-6,etc). The different virus dilutions were added to each well of the 24-well plate and incubated for 1 hour at 37°C, 5%CO₂. After that hour the supernatant containing the virus was removed from the ST monolayer and quickly an overlay AGAR was added onto the monolayer. The overlay AGAR was prepared using 1 part of 2X DMEM (Dubelco's Minimal Essential Medium) and 1 part of 1% purified AGAR in ddH₂O. After overlaying the cells the multi-well plate was kept for 15 minutes at room temperature to solidify the agarose and then was placed in a controlled incubator for 48 hours at 37°C, 5%CO₂.

In order to count the viral plaques the infected ST cells monolayer were fixed with 10% formol and stained with crystal violet 0.1% for 30 minutes. The well was washed with destilated water and dryed at room temperature to finally count the plaques to find the virus titer.

TGEV and rotavirus protein expression were analysed by standard immunofluorescence techniques.

### EXAMPLE 7

### Generation of rTGEV

The porcine transmissible gastroenteritis virus (TGEV) used belongs to the group of Purdue isolates, and was obtained in Indiana in 1946 (Doyle and Hutchings, 1946). The virus was adapted to grow in cell cultures (Haelterman and Pensaert, 1967), and was provided by E. H. Bohl (Ohio State University, Wooster Ohio). This TGEV isolate has been passaged in ST cells 115 times, and has been cloned five times consecutively in Dr. Luis Enjuanes laboratory (Centro Nacional de Biotecnologia, Madrid, Spain). The clone selected was labelled PUR46-CC120-MAD, abbreviated PUR46-MAD. This is an attenuated virus that grows well in cell cultures, and reaches titers between 10⁸ and 10⁹ PFU/mL.

rTGEV viruses were generated from pBAC-TGEV constructs containing the S gene from the virulent TGEV strain PUR-C11 (S_{C11}) as described (Alamazan et al., 2000 ; Gonzalez et al., 2002). Viruses containing the S gene (encoding the TGEV spike protein) from the attenuated strain PTV (S_{PTV}) were derived from the corresponding pBAC-TGEV vectors with S_{C11} by replacing this gene by the S_{PTV} of the respiratory strain.

### EXAMPLE 8

### Construction of a recombinant TGEV vector expressing rotavirus VLPs

In order to increase the cloning capacity of the TGEV single genome, the non-essential genes ORFs 3a and 3b were eliminated from the full-length cDNA clone, creating a deletion in the TGEV genome. The heterologous genes ORF2 and ORF6 encoding rotavirus structural proteins VP2 and VP6 were inserted in the cDNA construct, replacing the deleted TGEV ORFs 3a and 3b (Figure 3). The resultant cDNA encodes the recombinant virus rTGEV-S_{PTV}-*RS*-Δ3ab-VP2-VP6_{TRS22N} encoding the rotavirus ORF2 gene under the transcription-regulatory sequences of ORF 3a; and the ORF6 gene under the engineered TRS including the 5'TRS from N gene (TRS_{N}) that was inserted just downstream of the rotavirus ORF2 gene stop codon. The recombinant viral vector for rotavirus structural proteins VP2 and VP6 has been engineered to stably direct the expression of high levels of rotavirus Virus-like Particles (VLPs).

### Plasmid construct:

To facilitate the genetic manipulation of the viral genome, full-length cDNA clones were constructed by separating the contiguous genes and inserting unique restriction sites between each gene pair (Ortego et al. 2003). In order to increase the cloning capacity of the cDNA clone the non-essential genes 3a and 3b were deleted from the TGEV genome by removing the 884 base pair fragment *(Mlu* I - *Blp* I) from the intermediate plasmid pACNR-S_{PTV}-3EMN7C8-BGH.

The rotavirus ORF 2 gene which encodes the rotavirus VP2 structural protein has a size of 2.7 Kb. In order to clone this large gene in the rTGEV vector a cloning strategy was designed for the insertion of two restriction endonucleases sites *Mlu* I (5'-end) and *Blp* I (3'-end) in the ORF 2. In order to avoid nucleotide mutations the 5' -end and 3'-end of the ORF 2 was amplified separately by PCR assays.

The 5'-end of the ORF 2 was amplified by PCR from plasmid pcDNA-RF2 with a forward oligonucleotide (5'GC*GGATCC*ACGCGT*CATTACAGGTCCTGT*A*T*GGCGTACAGGAAACGTGGAGCGCG-3') (SEQ ID NO:6) containing the restriction sites *Bam* HI (bold and italic nucleotides), *Mlu* I (bold underlined nucleotides), the TRS from the 3a gene and a reverse oligonucleotide (5'CACAAGGATTCAAAATTGTCATG-3')(SEQ ID NO:7). The final PCR product was digested with restriction endonucleases *Bam* HI and *Bsg* I and cloned into the corresponding sites of plasmid pcDNA-RF2. This plasmid was labelled pcDNA-5*Mlu*RF2.

The 3'-end of the ORF 2 was amplified by PCR from plasmid pcDNA-RF2 with a forward oligonucleotide (5'-AAGC*CAACCCCACTGTG*GCTAAGCCCCCAATTCTGCAGATATCCATCAC-3') (SEQ ID NO:8) containing the restriction sites *Xcm* I (bold italic nucleotides), *Blp* I (bold, underlined nucleotides), and a reverse oligonocleotide (5'-GCGCCGTACAGGGCGCGTGGGG-3') (SEQ ID NO:9). The final PCR product was digested with restriction endonucleases *Xcm* I and *Bbs* I and cloned into the corresponding sites of plasmid pcDNA-5*Mlu*RF2. This plasmid was labelled pcDNA-5*Mlu*3*Blp*RF2.

The ORF 2 was digested with the restriction endonucleases *Mlu* I and *Blp* I from the plasmid pcDNA-5*Mlu*3*Blp*RF2 and cloned in the corresponding sites of plasmid pACNR-S_{PTV}-3EMN7C8-BGH by replacing the dispensable genes TGEV ORFs 3a and 3b. This plasmid was labelled pACNR-S_{PTV}-3EMN7C8-BGH-VP2.

An engineered TRS including 5 'TRS from the N gene TRS_{22N} (Sola et al, 2003) was used in order to construct a dicistronic TGEV vector for the expression of the rotavirus genes: ORF 2 and ORF 6. The ORF 6 encodes for VP6 rotavirus structural protein and was independently PCR amplified from plasmid pcDNA-RF6 using a forward oligonucleotide (5'CCGCC**GCTAAGC***AAAATTATTACATATGGTATAA*CTAAAGAAAATGGATGTCCTGTAC TCCTTGTCA-3') (SEQ ID NO:10) containing the restriction site *Blp* I (bold nucleotides), the core sequence (underlined nucleotides), and 22 nucleotides from the 5'-flanking sequences of the N gene (italic nucleotides) and a reverse oligonucleotide (5'GCGC**ATTTAAA**TCATTTGACAAGCATGCTTCTAATGG-3') (SEQ ID NO:11) including the restriction site *Swa* I (bold nucleotides). ORF6 gene was cloned at the *Mlu* I and *Swa* I sites of plasmid pACNR-S_{PTV}-3EMN7C8-BGH-VP2 generating an intermediate plasmid labelled pACNR-S_{PTV}-3EMN7C8-BGH-VP2-VP6_{TRS22N}. Fragment *Mlu* I - *Bam* HI including VP2 and VP6 gene was inserted in the corresponding sites of plasmid pBAC-TGEV-S_{PTV}-*RS* leading to plasmid pBAC-TGEV-S_{PTV}-*RS*-Δ3ab-VP2-VP6_{TRS22N} encoding rotavirus ORF2 gene under the ORF 3a transcription-regulatory sequences and ORF6 gene under the engineered TRS_{22N} (TRS_{N}) replacing the dispensable TGEV genes 3a and 3b.

### EXAMPLE 9

### Virus production

Swine testis cells (ST) were transfected with the cDNA encoding rTGEV-S_{PTV}-*RS*-Δ3ab-VP2-VP6_{TRS22N} genome, and infectious virus was recovered 48 h post-transfection. The virus production was amplified by passing the supernatants four times in cell cultures. As expected, no virus was recovered from the mock-transfected cultures. The cytopathic effect and plaque morphology produced by the rTGEV-S_{PTV}-*RS*-Δ3ab-VP2-VP6_{TRS22N} was identical to those of the parental viruses without heterologous genes. After four passages in cell culture, the recombinant viruses were cloned three times by plaque isolation steps.

### EXAMPLE 10

### Analysis of intracellular RNAs

Intracellular RNAs were isolated from cells infected with the rTGEV-S_{PTV}-*RS*-Δ3ab-VP2-VP6_{TRS22N} and were analysed by RT-PCR.

For detection of the subgenomic RNAs of the virus and heterologous rotavirus genes by obtaining complementary DNA, reverse transcriptase (RT) reactions have been performed with specific antisense (-) oligonucleotides that hybridised with each of the ORFs of each subgenomic RNA (Table 1). The reactions occurred in a volume of 25 µL at 42°C for one hour in the presence of 0.25 mM deoxynucleotide triphosphates (ATP, GTP, TTP, CTP), 1 mM DTT, 2.5 mM MgCl₂, PCR buffer and 6 U of reverse transcriptase SuperScript III RNase H⁻ purchased from Invitrogen.

Amplification of the DNA was accomplished by PCR using as a matrix 5 µL of the RT-PCR reactions. For amplification of the subgenomic RNAs, each of the antisense oligonucleotides described below (Table 1) were used together with an oligonucleotide having the leader sequence of TGEV (Leader oligonucleotide, 5'AGATTTTGTCTTCGGACACCAACTCG-3') (SEQ ID NO:12) . Because of the large size of the rotavirus ORF 2 mRNA it was impossible to amplify the entire mRNA, however a RT-PCR was designed to detect a fragment of the ORF 2 mRNA.

### Table 1

Oligonucleotides used in the RT-PCR reactions for detection of the sgmRNAs of TGEV and Rotavirus.

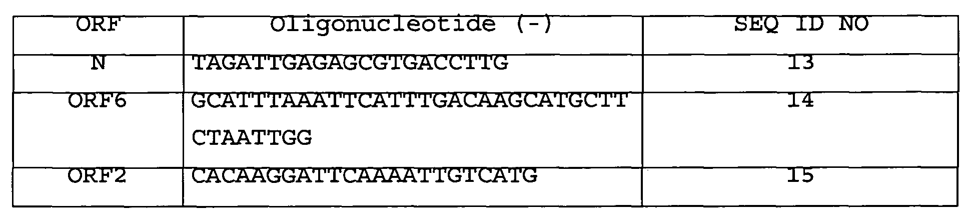

The PCR reactions occurred during 25-35 cycles at a Tm of 50°C and 60 s of elongation at 72°C.

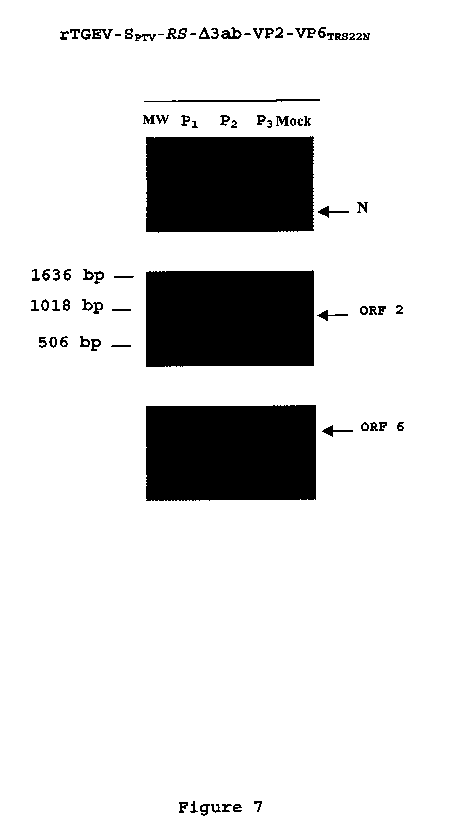

The products of the RT-PCR reactions were analyzed by agarose gel electrophoresis. The major products of the RT-PCR reactions showed the expected size for N of the viral and heterologous rotavirus mRNAs (Figure 7).

### EXAMPLE 11

### Analysis of proteins

### Electrophoresis of proteins in polyacrylamide gels (SDS-PAGE):

All the protein samples were analysed in 10% polyacrylamide gels in the presence of sodium dodecyl sulphate (SDS-PAGE electrophoresis).

### Transfer of proteins to nitrocellulose membranes and immunodetection with specific antibodies (transfer and immunodetection or Western blot type transfer):

For the detection of the proteins by immunodetection, the proteins separated by SDS-PAGE electrophoresis were transferred to nitrocellulose membranes with a Mini Protean II electrotransfer apparatus (Bio-Rad) at 150 mA for 1 h in transfer buffer (25 mM Tris-192 mM glycine, 20% methanol, pH 8.3). The membranes were blocked for 2 h with 5% powdered skimmed milk (Nestlé) in TBS buffer (20 mM Tris-HCl, pH 7.5, 150 mM NaCl) at room temperature. Membranes were incubated with specific MAbs for the N protein of TGEV or specific MAbs against VP2 and VP6 rotavirus proteins. The bounded antibody was detected with goat antibodies specific for mouse IgG immunoglobulins, conjugated with radish peroxidase, using the ECL chemoluminescence system (Amersham Pharmacia Biotech).

### Analysis of expressed proteins by western blot:

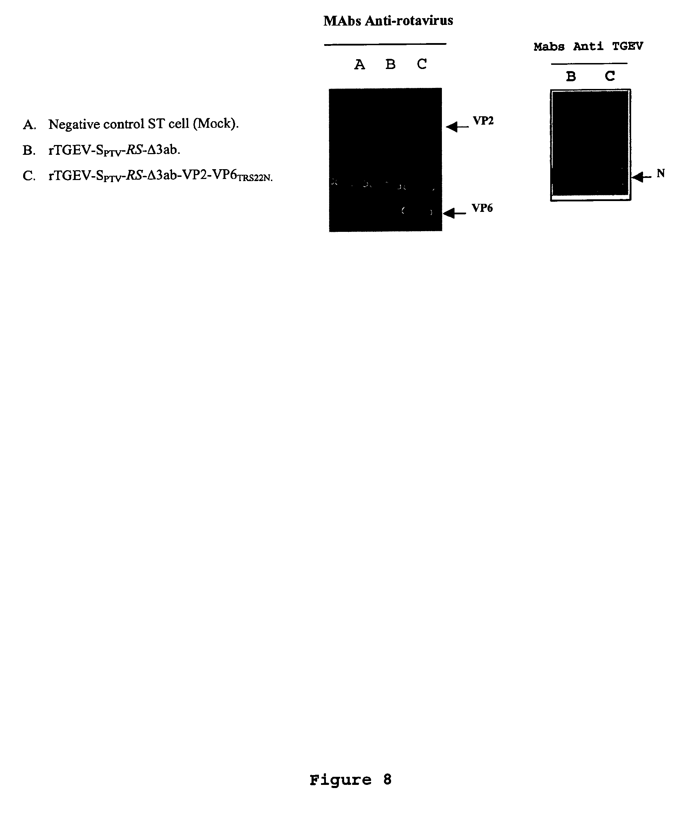

The production of viral and heterologous VP2 and VP6 rotavirus structural proteins at 16 h post-infection was studied by western blotting using MAbs specific for these proteins (Figure 8).

### Antibodies:

The specificity of MAbs 5B.H1, 9D.B4, 3D.E3, 3B.B3, 3D.C10, 25.22 and 1A6 has been characterized previously (Charley and Laude, 1988; Jiménez et al., 1986; Laude et al., 1992; Martín-Alonso et al., 1992; Risco et al., 1995; Suñe et al., 1990; Wesley et al., 1988; Woods et al., 1987). MAbs 9D.B4, 3B.B3 and 3D.E3 specifically recognize the carboxy terminal of the M protein of TGEV, and MAbs 25.22 and 1A6 are specific for the amino terminal (Charley and Laude, 1988; Laude et al., 1992; Wesley et al., 1988; Woods et al., 1987). MAbs 3D.C10 and 5BH1 recognize the N and S proteins of TGEV, respectively (Jiménez et al., 1986; Martin-Alonso et al., 1992). Monoclonal antibodies against rotavirus proteins VP2 and VP6 were characterized and gently provided by Dr. Jean Cohen (INRA, jouy-en-Josas, France).

The F fraction (ab)₂ of a fluresceine-conjugated goat anti-mouse immunoglobulin antibody was acquired from Cappel.

### EXAMPLE 12

### Analysis of expressed proteins by confocal microscopy

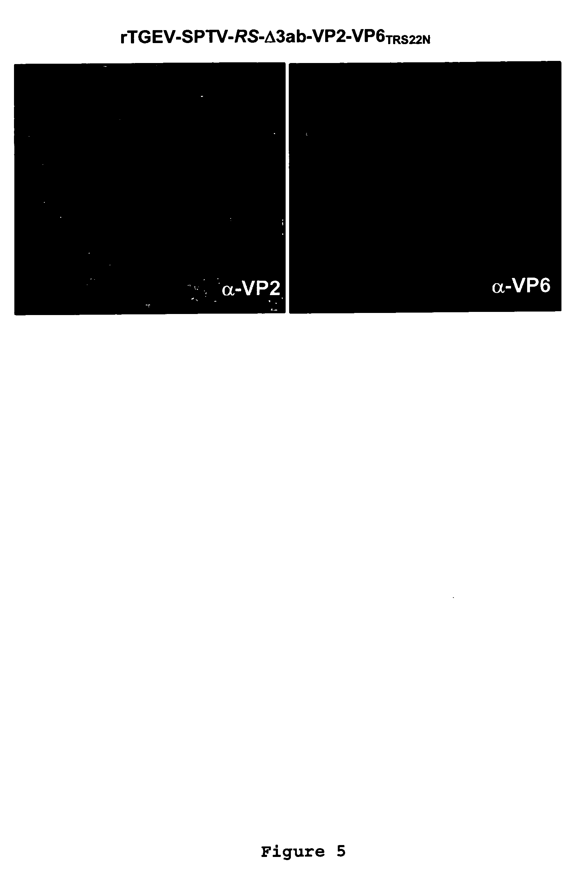

The expression of VP2 and VP6 proteins at 16 h post-infection was observed in 70% of the cells infected with rTGEV-S_{PTV}-*RS-*Δ3ab-VP2-VP6_{TRS22N} by fluorescence microscopy using specific MAbs against rotavirus heterologous proteins (Figure 5).

Cells were grown on 12-mm-diameter glass coverslips to 60% confluence. For immunodetection, cells were washed with phosphate-buffered saline (pH 7.4) containing 1% bovine serum albumin (PBS-BSA), fixed with 4% paraformaldehyde for 30 minutes at room temperature and incubated for 90 minutes with specific MAb and specific MAb against rotavirus structural proteins VP2 and VP6 (1:250 dilution in PBS-BSA containing 0,1% Saponin [Superfos Biosector, Vedback, Denmark]).

The cells were washed three times with PBS-BSA and incubated with Alexa-488-conjugated anti-mouse immunoglobulin G (1:500 dilution Cappel) for 30 minutes at room temperature. The coverslides were washed five times with PBS-BSA, mounted on glass slides, and analysed with a confocal microcopy.

### EXAMPLE 13

### Assembly of rotavirus virus-like-particles

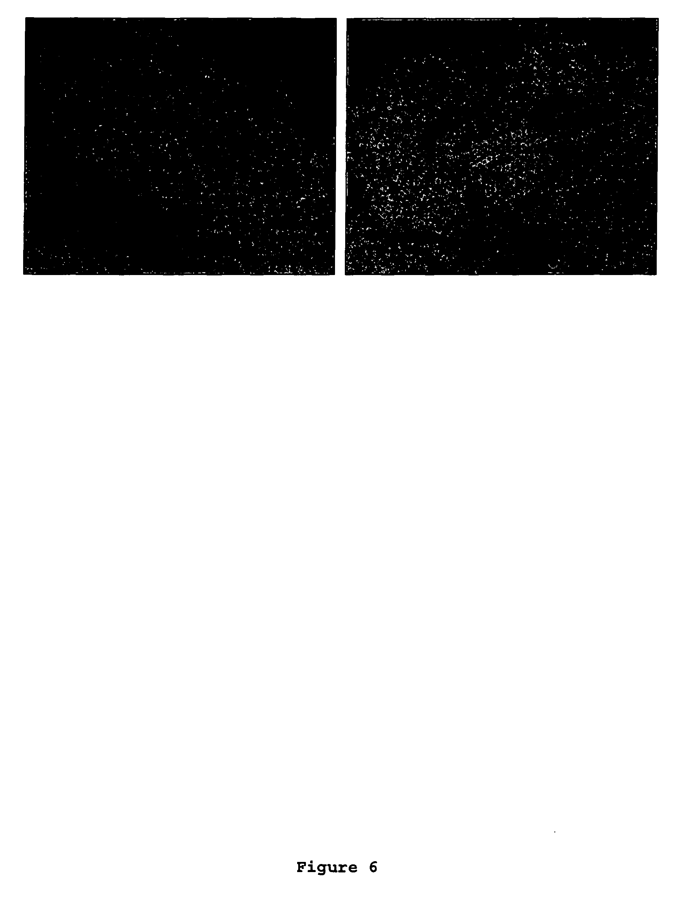

The assembly of rotavirus VP2-VP6 Virus-like Particles (VLPs) was analysed in ST cells infected with the recombinant TGEV virus by electron microscopy (Figure 6).

ST cells monolayers were infected with rTGEV-S_{PTV}-*RS*-Δ3ab-VP2-VP6_{TRS22N} . The cells were fixed *in situ* at 16 h post infection with a mixture of 2% glutaraldehyde and 1% tannic acid in 0,4 M HEPES buffer (pH 7.2) for 2 h at room temperature. Fixed monolayers were removed from dishes in the fixative and transferred to Eppendorf tubes. After centrifugation, the cells were washed with HEPES buffer and the pellets were processed for embedding in EML-812 (Taab Laboratories, Berkshire, United Kingdom) as it was described previously (Risco,et al. 1998). Cells were post-fixed with a mixture of 1% osmium tetroxide and 0.8% potassium ferricyanide in destilled water for 1 h at 4°C. After four washes with HEPES buffer, samples were incubated with 2% uranyl acetate, washes again, and dehydrated in increasing acetone concentrations (50, 70, 90 and 100%) for 15 min at 4°C. Infiltration in the resin EML-812 was done at room temperature for 1 day. Polymerization of filtrated samples was done at 60°C for two days. Ultrathin (50-to 60-nm-thick) sections of the samples were stained with saturated uranyl acetate and lead citrate by standard procedures.

The electron microscopy photograph of infected ST cells with rTGEV-S_{PTV}-RS-Δ3ab-VP2-VP6_{TRS22N} showed rounded structures similar in size and appearance to Rotavirus VLPs obtained by other in vitro expression systems indicating that VP2/VP6-VLPs were correctly assembled.

No similar structures were observed in the negative control (infected ST cells with TGEV wt).

In addition, several experiments were performed that confirm the transcription and transduction of VP6 and VP2 rotavirus genes in rTGEV-S_{PTV}-RS-Δ3ab-VP2-VP6_{TRS22N} infections, by RT-PCR, immunofluorescence and Western blot assays.

TGEV virus particles comprising the above recombinant viral vector, rTGEV-S_{PTV}-*RS*-Δ3ab-VP2-VP6_{TRS22N'} encapsulated by TGEV coat proteins were deposited according to the provisions of the Budapest Treaty with the Institute Pasteur (Paris, France) on August 31, 2004 under the Registration Number CNCM I-3289.

### Bibliography:

Alamazan, F., Gonzales J.M., Penzes Z., Izeta A., Calvo E., Plana-Duran J., and Luis Enjuanes (2000). Proc. Natl. Acad. Sci. USA **97**: 5516-5521.
Alonso, S., Izeta A., Sola I., and Enjuanes L. (2002a). Transcription regulatory sequences and mRNA expression levels in the coronavirus transmissible gastroenteritis virus. J. Virol. **76**:1293-1308
Alonso, S., Sola, I., Teifke, J., Reimann, I., Izeta, A., Balach, M., Plana-Durán, J., Moormann, R. J. M., and Enjuanes, L. (2002b). In vitro and in vivo expression of foreign genes by transmissible gastroenteritis coronavirus-derived minigenomes. *J. Gen. Virol.* **83**, 567-579.
Bullock, W., Fernández, J.M. and Short, J.M. (1987). XL1-Blue: a high efficiency plasmid transforming recA *E.coli* strain with P-galactosidadse selection. Biotechniques **8**:26-27.
Chang, K.-Y., and Tinoco, I. (1994). Characterization of a "kissing" hairpin complex derived from the human immunodeficiency virus genome. *Proc. Natl. Acad. Sci. USA* **91**, 8705-8709.
Charley, B. and Laude H. (1988). Induction of alpha-interferon by transmissible gastroenteritis coronavirus: Role of transmembrane glycoprotein El. J. Virol **62**:8-10.
Delmas B, Gelfi J., Kut E., Sjostrom H., Noren O., Laude H. (1994). Determinants essential for the transmissible gastroenteritis virus-receptor interaction reside within a domain of aminopeptidase-N that is distinct from the enzymatic site. J Virol. **68(8)**:5216-24.
Doyle, L.P. and Hutchings, L.M. (1946). A transmissible gastroenteritis in pigs. J. Amer. Vet. Med. Assoc. **108**:257-259.
Enjuanes L., et al. (2003). Virus based vectors for gene expression in mammalian cells; Coronaviruses; in *Gene transfer and expression in mammalian cells;* Ed. S.C. Makrides, p. 151-158.
Gonzalez, J.M., Penzes Z., Almazan F., Calvo E., and Luis Enjuanes (2002). Stabilization of a full-length infectious cDNA clone of transmissible gastroenteritis coronavirus by the insertion of an intron. J. Virol. **76**: 4655-4661.
Haelterman E.O. and Pensaert M.B. (1967). Pathogenesis of transmissible gastroenteritis of swine. Proc. 18th World Vet. Congress **2**:569-572.
Hanahan D., Jessee J., Bloom F.R. (1991). Plasmid transformation of Escherichia coli and other bacteria. Methods Enzymol. **204**: 63-113.
Izeta, A., Smerdou, C., Alonso, S., Penzes, Z., Mendez, A., Plana-Durán, J., and Enjuanes, L. (1999). Replication and packaging of transmissible gastroenteritis coronavirus-derived synthetic minigenomes. *J*. *Virol.* **73**, 1535-1545.
Jimenez G., Correa I., Melgosa M.P., Melgosa M.P., Bullido M.J., Enjuanes L. (1986). Critical epitopes in transmisible gastroenteritis virus neutralization. J. Virol **60**:131-139.
Kim, Y., Kyeong-Ok, Kim W., Saif L. J. (2002). Production of Hybrid Double or Triple-Layered Virus Like Particles of Group A and C Rotaviruses using a Baculovirus expression system. Virology **302**: 1-8.
Kiyono et al. (1996). Mucosal Vaccines. Academic Press, New York.
Laude, H., Rasschaert D., Delmas B., Godet M., Gelfi J., and Bernard C. (1990). Molecular biology of transmissible gastroenteritis virus. Vet. Microbiol. **23**: 147-154.
Laude H., Gelfi J., Lavenant L., Charley B. (1992). Single amino acid changes in the viral glycoprotein M affect induction of alpha interferon by the coronavirus transmissible. J. Virol. **66**:743-749.
Martin-Alonso J.M., Balbin M., Garwes D. J., Enjuanes L., Gascon S., Parra F. (1992). Antigenic structure of transmisible gastroenteritis virus nucleoprotein. Virology **188**: 168-174
McClurkin, A.W. and Noman, J.O. (1966). Studies on transmissible gastroenteritis of swine. II. Selected characteristics of a cytopathogenic virus common to five isolates from transmissible gastroenteritis. Can. J. Comp. Med. Vet. Sci. **30**:190-198.
Ortego J., Escors D., Laude H., and Luis Enjuanes (2002). Generation of a replication-competent, propagation deficient virus vector based on the transmissible gastroenteritis coronavirus genome. J.Virol. **76**:11518-11529.
Ortego J., Sola I., Almazan F., Ceriani J.E., Riquelme C., Balach M., Plana-Duran J. and Luis Enjuanes (2003). Transmissible gastroenteritis coronavirus gene 7 is not essential but influences *in vivo* replication and virulence. Virology **308**: 13-22.
Penzes Z., Gonzales J.M., Calvo E., Izeta A., Smerdou C., Mendez A., Sanches C.M., Sola I., Almazan F., Enjuanes L. (2001). Complete genome sequence of Transmissible Gastroenteritis Coronavirus PUR46-MAD clone and evolution of the purdue virus cluster. Virus Genes **23**:1, 105-118.
Risco C., Anton I., Suné C., Pedregosa A.M., Martí-Alonso J.M., Parra F., Carrascosa J.L., Enjuanes L. (1995). Membrane protein molecules of transmissible gastroenteritis coronavirus also expose the carboxy-terminal region on the external surface of the virion. J. Virol. **69**:5269-5277.
Risco, C., Muntión M., Enjuanes L., Carrascosa J.L. (1998). Two types of virus-related particles during transmisible gastroenteritis virus morphogenesis. J. Virol 72:4022-4031.
Sambrook et al. (1989). Molecular Cloning: A Laboratory Manual. Ed Cold Spring Harbor Laboratory.
Sánchez, C. M., Izeta, A., Sánchez-Morgado, J. M., Alonso, S., Sola, I., Balasch, M., Plana-Durán, J. and Enjuanes, L. (1999). Targeted recombination demonstrates that the spike gene of transmissible gastroenteritis coronavirus is a determinant of its enteric tropism and virulence. J. Virol. 73:7607-7618.
Sawicki & Sawicki (1990). Coronavirus transcription: subgenomic mouse hepatitis virus replicative intermediates function in RNA synthesis. J Virol. **64**(3):1050-6.
Schwartz-Cornil I., Benureau Y., Greenberg H., Hendrickson B.A., Cohen J. (2002). Heterologous protection induces by the inner capsid proteins of rotavirus requires transcytosis of mucosal immunoglobulins. J. Virol. **76**: 8110-8117.
Sethna, P. B., Hung, S.-L., and Brian, D. A. (1989). Coronavirus subgenomic minus-strand RNAs and the potential for mRNA replicons. *Proc. Natl. Acad. Sci. USA* **86**, 5626-5630.
Siddell, S. G. (1995). "The *Coronaviridae."* The Viruses (H. Fraenkel-Conrat, and R. R. Wagner, Eds.) Plenum Press, New York.
Sola I, Alonso S., Zúñiga S., Balasch M., Plana-Duran J., Enjuanes L. (2003). Engineering the transmissible gastroenteritis virus genome as an expression vector inducing lactogenic immunity. J. Virol. **77**:4357-4369.
Suñe C., Jimenez G., Correa I., Bullido M.J., Gebauer F., Smerdou C., Enjuanes L. (1990). Mechanisms of transmissible gastroenteritis coronavirus neutralization. Virology **177**:559-569.
Toka, F.N. et al. (2004). Molecular adjuvants for mucosal immunity. Immunol Rev. **199**:100-112.
van der Most, R. G., and Spaan, W. J. M. (1995). Coronavirus replication, transcription, and RNA recombination. *In* "The Coronaviridae" (S. G. Siddell, Ed.), pp. 11-31. Plenum Press, New York.
Wesley R.D., Woods R.D., Correa I., Enjuanes L. (1988). Lack of protection in vivo with neutralization antibodies to transmissible gastroenteritis virus. Vet. Microbiol. **18**:197-208.
Woods, RD., Wesley, RD. and Kapke, P.A. (1987). Complement-dependent neutralization of transmissible gastroenteritis virus by monoclonal antibodies. Adv. Exp. Med. Biol. **218**:493-500.

## Claims

1. Nucleic acid comprising:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding one or more proteins of a different virus, which is not FMDV, wherein the one or more proteins are capable of associating into a virus-like particle (VLP) that does not contain any infectious nucleic acid.

2. Nucleic acid according to claim 1, wherein the nucleic acid encodes a TGEV replicase and a sequence encoding the TGEV N protein.

3. Nucleic acid according to claim 1 or 2, wherein the nucleic acid further encodes SEQ ID NO: 16 or a sequence having a homology of at least 60% to SEQ ID NO.:16.

4. Nucleic acid according to any one of claims 1 to 3, wherein the replication competent TGEV vector is not infectious.

5. Nucleic acid according to any one of claims 1 to 3, wherein the replication competent TGEV vector is infectious.

6. Nucleic acid according to claim 5, wherein the nucleic acid further comprises one or more of the following TGEV genes: S, E, M and/or N or sequences having a homology of at least 60% to the given sequence.

7. Nucleic acid according to claim 5 or 6, wherein the TGEV infectious viral particles obtainable from the association of TGEV proteins and the nucleic acid sequences are attenuated viral particles.

8. Nucleic acid according to any of claims 1 to 7, wherein the nucleic acid sequences **characterized in** (b) of claim 1 are derived from rotavirus, SARS virus, PCV or parvovirus.

9. Nucleic acid according to one of claims 1 to 8, wherein the virus-like particles of rotavirus, SARS virus PCV or parvovirus are capable of generating an immune response in a mammal.

10. Nucleic acid according to any of claims 1 to 9, wherein the nucleic acid sequences encoding rotavirus proteins are of human and/or animal origin and comprise sequences encoding at least two of the following proteins:
VP2 (SEQ ID NO:1),
VP4 (SEQ ID NO:2),
VP6 (SEQ ID NO:3),
VP7 (SEQ ID NO:4)
or sequences having a homology of at least 60 % to the SEQ ID NO:1 to 4.

11. Nucleic acid comprising:
(a) sequences of a replication competent but non-infectious transmissible gastroenteritis virus (TGEV), which encode a TGEV replicase under the control of expression regulatory sequences so that expression of the replicase in a cell containing the nucleic acid will initiate replication of the nucleic acid and thus increase the number of nucleic acids in the cell and a sequence encoding the TGEV N protein; and
(b) at least two of the following rotavirus sequences:
VP2 (SEQ ID NO:1),
VP4 (SEQ ID NO:2),
VP6 (SEQ ID NO:3),
VP7 (SEQ ID NO:4) or sequences having a homology of at least 60 % to the SEQ ID N0:1 to 4.

12. Nucleic acid comprising:
(a) sequences of a replication competent and infectious transmissible gastroenteritis virus (TGEV), which encode a TGEV replicase under the control of expression regulatory sequences so that expression of the replicase in a cell containing the nucleic acid will initiate replication of the nucleic acid and thus increase the number of nucleic acids in the cell and a sequence encoding the TGEV N protein; and
(b) at least two of the following rotavirus sequences:
VP2 (SEQ ID NO:1),
VP4 (SEQ ID NO:2),
VP6 (SEQ ID NO:3),
VP7 (SEQ ID NO:4) or sequences having a homology of at least 60 % to the SEQ ID NO:1 to 4.

13. Nucleic acid according to any of claims 1 to 12, wherein the nucleic acid sequences encoding rotavirus proteins further comprise sequences encoding fusion proteins.

14. Nucleic acid according to claim 13, wherein the nucleic acid sequence encodes a rotavirus VP8-VP2 fusion protein (SEQ ID N0:5) or a sequence having a homology of at least 60 % to the SEQ ID NO:5.

15. Nucleic acid comprising:
(a) sequences of a replication competent but non-infectious transmissible gastroenteritis virus (TGEV), which encode a TGEV replicase under the control of expression regulatory sequences so that expression of the replicase in a cell containing the nucleic acid will initiate replication of the nucleic acid and thus increase the number of nucleic acids in the cell and a sequence encoding the TGEV N protein; and
(b) two or all of the following SARS-CoV sequences:
S (SEQ ID NO:17),
M (SEQ ID NO:18),
E (SEQ ID NO:19), or sequences having a homology of at least 60 % to the SEQ ID NO:17 to 19.

16. Nucleic acid comprising:
(a) sequences of a replication competent and infectious transmissible gastroenteritis virus (TGEV), which encode a TGEV replicase under the control of expression regulatory sequences so that expression of the replicase in a cell containing the nucleic acid will initiate replication of the nucleic acid and thus increase the number of nucleic acids in the cell and a sequence encoding the TGEV N protein; and
(b) two or all of the following SARS-CoV sequences:
S (SEQ ID NO:17),
M (SEQ ID NO:18),
E (SEQ ID NO:19), or sequences having a homology of at least 60 % to the SEQ ID NO:17 to 19.

17. Nucleic acid according to any of claims 1 to 16, which is DNA or RNA.

18. Recombinant RNA encoded by a nucleic acid according to any of claims 1 to 17.

19. Vector comprising a nucleic acid according to one of claims 1 to 18.

20. Vector according to claim 19, wherein the vector is a cDNA vector.

21. Vector according to claim 20, wherein the vector is a BAC-TGEV^{FL} vector.

22. Vector according to any of claims 19 to 21, wherein the vector is capable of replicating the nucleic acid within a host cell.

23. Host cell comprising a vector according to one of claims 19 to 22.

24. Host cell according to claim 23, wherein the cell is a bacterial cell, a yeast cell, an insect cell,an animal cell or a human cell.

25. Host cell according to claim 24, wherein the cell is a porcine swine testis cell line, such as the cell line deposited under ATCC CRL-1746.

26. Virus particle comprising a nucleic acid according to any of claims 1 to 17 and at least one TGEV coat protein, wherein the virus particle is preferably the virus particle deposited under CNCM 1-3289 .

27. Virus particle according to claim 26, comprising all TGEV coat proteins of the native TGEV virus particle.

28. Method of preparing virus-like particles, which are not FMDV virus-like particles, that do not contain any infectious nucleic acid, comprising steps, wherein a nucleic acid sequence according to any of claims 1-17 is expressed in a host cell in cell culture and the virus-like particles are isolated from the medium and/or from the host cells.

29. Pharmaceutical preparation comprising a nucleic acid according to one of claims 1 to 17, a viral RNA or vector according to one of claim 18 to 22 or a host cell according to one of claims 23 to 25 or a virus particle according to claim 26 or 27.

30. Pharmaceutical preparation according to claim 29 further comprising a pharmaceutically acceptable carrier, excipient and/or adjuvants.

31. Vaccine capable of protecting an animal or a human against a disease caused by an infectious virus comprising a nucleic acid according to one of claims 1 to 17, a viral RNA or vector according to one of claim 18 to 22, a host cell according to one of claims 23 to 25 or a virus particle according to claim 26 or 27.

32. Vaccine according to claim 31 further comprising a pharmaceutically acceptable carrier, excipient and/or adjuvants.

33. Vaccine according to claim 31 or 32, wherein the vaccine is suitable for vaccinating an animal, such as a human, a ruminant, a swine or a bird.

34. Vaccine capable of protecting a human against a disease caused by rotavirus infection comprising a virus particle, wherein the virus particle comprises a nucleic acid according to claim 11 or 12 and at least one or all of the TGEV coat proteins.

35. Vaccine capable of protecting a human against a disease caused by SARS―CoV infection comprising a virus particle, wherein the virus particle comprises a nucleic acid according to claim 15 or 16 and at least one or all of the TGEV coat proteins.

36. Vaccine according to any of claims 31 to 35, wherein the vaccine is capable of inducing both a systemic immune response and a mucosal immune response against infectious viral agents.

37. Vaccine according to any of claims 31 to 36, wherein the infectious agent is, rotavirus, PCV, SARS virus or TGEV.

38. Use of a vaccine according to any of claims 31 to 37 for the protection of animals against viral infection, wherein the vaccine is administered by intramuscular, intravenous or oronasal administration.

39. Method for diagnosing whether an animal or a human is infected with a virus, which is not FMDV, or has been vaccinated using a vaccine according to one of claims 31 to 38 comprising steps, wherein the diagnosis uses antibodies specific for proteins of the wild type virus but not expressed by the vaccine strain.
